# EUROPÄISCHE PATENTSCHRIFT

(11) **EP 0 540 472 B1**
(45) Veröffentlichungstag und Bekanntmachung des Hinweises auf die Patenterteilung: **09.07.1997**
(21) Anmeldenummer: 92810822.4
(22) Anmeldetag: 23.10.1992
(51) Int. Cl.: C07D 213/75, A01N 43/00, C07D 215/38, C07D 223/12, C07D 239/42, C07D 241/20, C07D 249/14, C07D 261/14, C07D 263/58, C07D 277/46, C07D 277/58, C07D 277/82, C07D 285/16, C07D 285/12, C07D 307/32, C07D 413/04

(54) **Buttersäurederivate, Verfahren zu ihrer Herstellung und ihre Verwendung als Schädlingsbekämpfung**
Butyric acid derivatives, process for their preparation and their use as pesticides
Dérivés de l'acide butyrique, procédé pour leur préparation et leur utilisation comme pesticides

(30) Priorität: 01.11.1991 CH 3195/91
(43) Veröffentlichungstag der Anmeldung: 05.05.1993
(73) Patentinhaber: Novartis AG, 4058 Basel (CH)
(72) Erfinder: Maienfisch, Peter, Dr., CH-4118 Rodersdorf (CH); Böger, Manfred, W-7858 Weil am Rhein 5 (DE); Pitterna, Thomas, Dr., CH-4053 Basel (CH)

(56) Entgegenhaltungen:
- EP-A- 0 413 666
- EP-A- 0 472 497
- US-A- 4 950 666

## Beschreibung

Die vorliegende Erfindung betrifft neue Derivate der 4-Chlor-4,4-difluor-buttersäure, Verfahren zu ihrer Herstellung, Schädlingsbekämpfungsmittel, welche diese Verbindungen enthalten, sowie ihre Verwendung zw Kontrolle von Schädlingen.

Die erfindungsgemässen 4-Chlor-4,4-difluor-buttersäure-amide, -ester und -thioester entsprechen der Formel 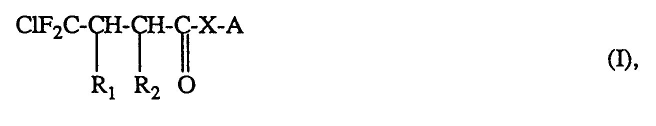 worin
A einen substituierten oder unsubstituierten, über ein Kohlenstoffatom an X gebundenen, aromatischen oder nicht-aromatischen, mono- oder bicyclischen, heterocyclischen Rest;
R₁ und R₂ unabhängig voneinander Wasserstoff oder C₁-C₆-Alkyl;
X NR₃, O oder S; und
R₃ Wasserstoff oder C₁-C₄-Alkyl
bedeuten.

In EP-A-0 413 666 und US-4,950,666 werden 4-Chlor-4,4-difluor-buttersäure-Derivate als Wirkstoffe in Schädlingsbekämpfungsmitteln vorgeschlagen. Die biologischen Eigenschaften der in diesen Publikationen beschriebenen Verbindungen vermögen auf dem Gebiet der Schädlingsbekämpfung jedoch nicht voll zu befriedigen, weshalb das Bedürfnis besteht, weitere Verbindungen mit schädlingsbekämpfenden Eigenschaften zur Verfügung zu stellen, wobei diese Aufgabe erfindungsgemäss durch die Bereitstellung der vorliegenden Verbindungen I gelöst wird.

Die erfindungsgemässen Verbindungen I schliessen auch, insbesondere agrochemisch verträgliche, Säureadditionssalze ein. Beispiele geeigneter (anorganischer oder organischer) Säuren sind Salzsäure, Bromwasserstoffsäure, Schwefelsäure, Phosphorsäure, Salpetersäure, Säuren mit gleichem Zentralatom und höherer oder niedrigerer Oxidationsstufe, wie Perchlorsäure, salpetrige Säure oder phosphorige Säure, Essigsäure und Bernsteinsäure.

Bevorzugte Ausführungsformen im Rahmen der Erfindung sind
(1) Eine Verbindung der Formel I, worin R₁ Wasserstoff ist;
(2) Eine Verbindung der Formel I, worin R₂ Wasserstoff ist;
(3) Eine Verbindung der Formel I, worin X NR₃ oder S und R₃ Wasserstoff oder C₁-C₄-Alkyl, insbesondere X NH oder S, vorzugsweise X NH, ist;
(4) Eine Verbindung der Formel I, worin der, über ein Kohlenstoffatom an X gebundene, Rest A ausgewählt ist aus der Gruppe von Resten, bestehend aus 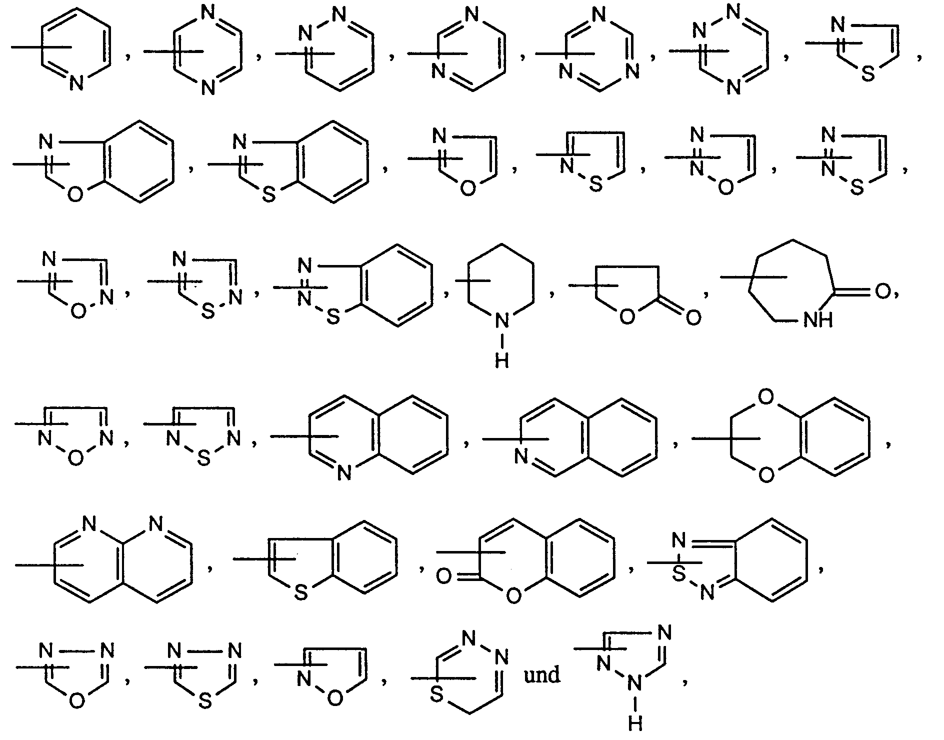 und unsubstituiert ist oder einen, zwei, drei oder vier Substituenten, ausgewählt aus der Gruppe, bestehend aus Halogen, C₁-C₆-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, Nitro, Cyano, C₁-C₄-Alkoxycarbonyl, Di-C₁-C₄-alkylamino, Phenyl, Benzyl, Pyridyl, Thienyl und durch Halogen, C₁-C₄-Alkyl, C₁-C₄-Halogenalkyl mit 1 bis 9 Halogenatomen, C₁-C₄-Alkoxy, Nitro oder Cyano einfach substituiertem Phenyl, Benzyl, Pirdyl oder Thienyl, trägt,
   insbesondere eine Verbindung der Formel I, worin der, über ein Kohlenstoffatom an X gebundene, Rest A ausgewält ist aus der Gruppe von Resten, bestehend aus 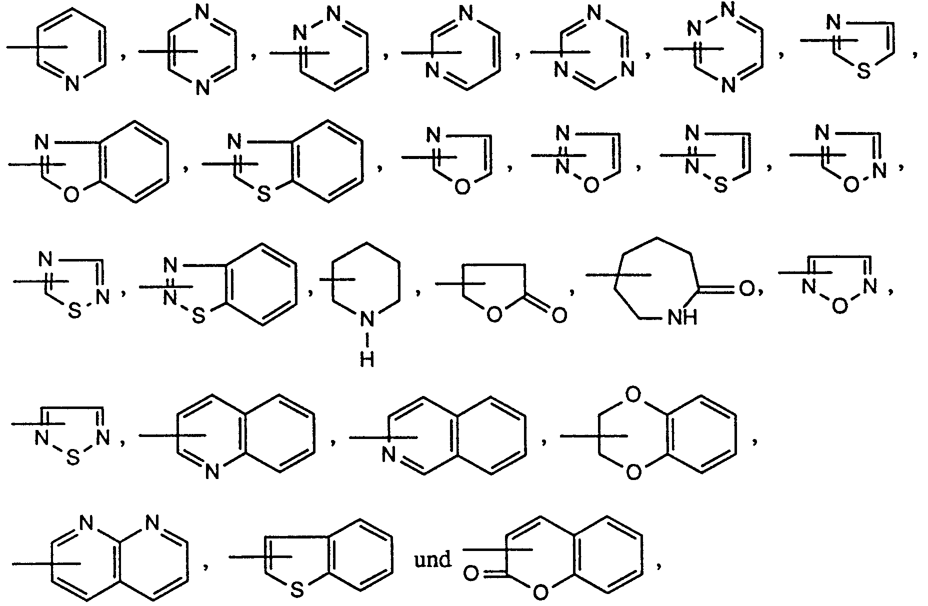 und unsubstituiert ist oder einen oder zwei Substituenten, ausgewählt aus der Gruppe, bestehend aus Halogen, C₁-C₆-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, Nitro, Cyano, C₁-C₄-Alkoxycarbonyl, Di-C₁-C₄-alkylamino, Phenyl, Benzyl, Pyridyl, Thienyl und durch Halogen, C₁-C₄-Alkyl, C₁-C₄-Halogenalkyl mit 1 bis 9 Halogenatomen, C₁-C₄-Alkoxy, Nitro oder Cyano einfach substituiertem Phenyl, Benzyl, Pyridyl oder Thienyl, trägt, insbesondere eine Verbindung der Formel I, worin der, über ein Kohlenstoffatom an X gebundene, Rest A ausgewählt ist aus der Gruppe von Resten, bestehend aus 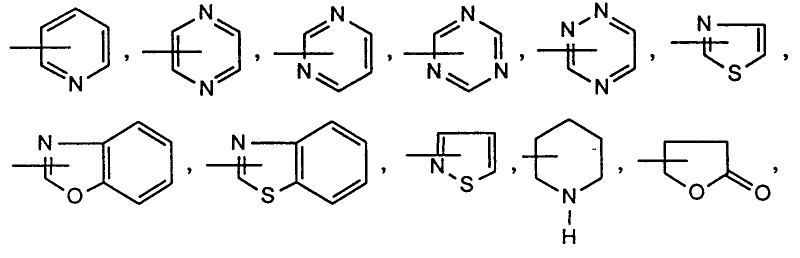 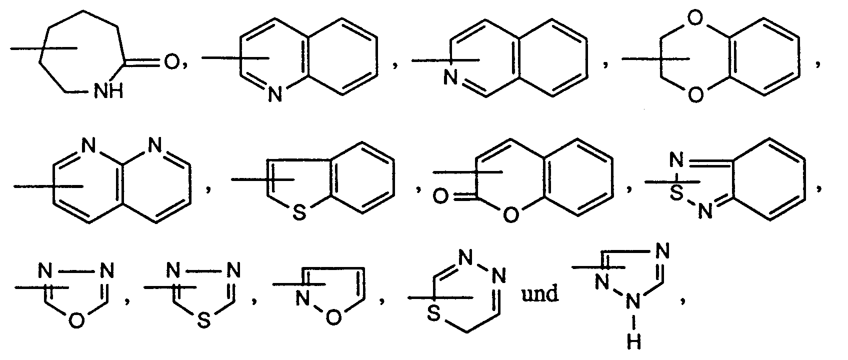 und unsubstituiert ist oder einen, zwei, drei oder vier Substituenten, ausgewählt aus der Gruppe, bestehend aus Halogen, C₁-C₆-Alkyl, C₁-C₄-Halogenalkyl mit 1 bis 9 Halogenatomen, C₁-C₄-Alkoxy, Nitro, C₁-C₄-Alkoxycarbonyl, Di-C₁-C₄-alkylamino, Phenyl, Benzyl, Pyridyl und Thienyl, trägt,
   insbesondere eine Verbindung der Formel I, worin der, über ein Kohlenstoffatom an X gebundene, Rest A ausgewählt ist aus der Gruppe von Resten, bestehend aus 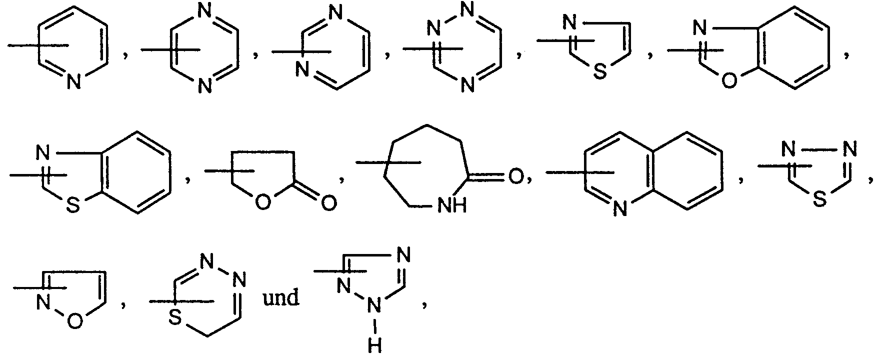 und unsubstituiert ist oder einen, zwei oder drei Substituenten, ausgewählt aus der Gruppe, bestehend aus Halogen, C₁-C₆-Alkyl, Nitro, Phenyl, Pyridyl und Thienyl, trägt, insbesondere eine Verbindung der Formel I, worin der, über ein Kohlenstoffatom an X gebundene, Rest A ausgewählt ist aus der Gruppe von Resten, bestehend aus 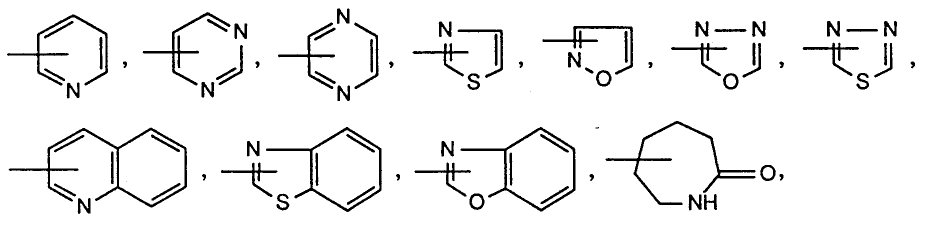 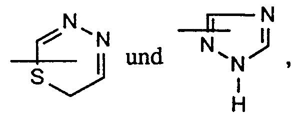 und unsubstituiert ist oder einen, zwei oder drei Substituenten, ausgewählt aus der Gruppe, bestehend aus Halogen, Methyl, Pyridyl und Thienyl, trägt,
   insbesondere eine Verbindung der Formel I, worin der, über ein Kohlenstoffatom an X gebundene, Rest A ausgewählt ist aus der Gruppe von Resten, bestehend aus 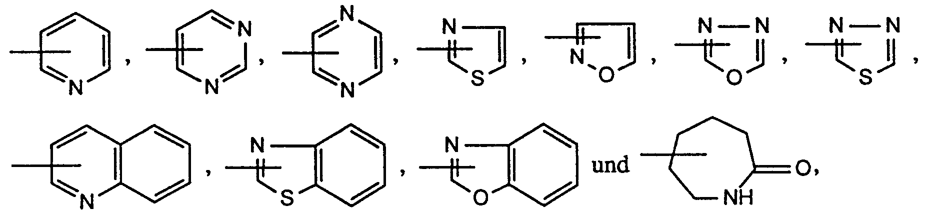 und unsubstituiert oder durch Halogen, Methyl, Pyridyl oder Thienyl monosubstituiert ist, insbesondere eine Verbindung der Formel I, worin der Rest A ausgewählt ist aus der Gruppe von Resten, bestehend aus Pyrid-2-yl, Pyrid-3-yl, Pyrimidin-2-yl, Pyrazin-2-yl, Thiazol-2-yl, Isoxazol-3-yl, 1,3,4-oxadiazol-2-yl, 1,3,4-Thiadiazol-2-yl, Benzoxazol-2-yl, Benzothiazol-2-yl und Chinolin-3-yl, und unsubstituiert oder durch Halogen, Methyl, Pyridyl oder Thienyl monosubstituiert ist,
   insbesondere eine Verbindung der Formel I, worin der Rest A unsubstituiertes Pyrid-2-yl oder unsubstituiertes Thiazol-2-yl ist;
(5) Eine Verbindung der Formel I, worin R₁ Wasserstoff ist, R₂ Wasserstoff ist, X NH oder S ist und der Rest A ausgewählt ist aus der Gruppe von Resten, bestehend aus Pyrid-2-yl, Pyrid-3-yl, Pyrimidin-2-yl, Pyrazin-2-yl, Thiazol-2-yl, Isoxazol-3-yl, 1,3,4-Oxadiazol-2-yl, 1,3,4-Thiadiazol-2-yl, Benzoxazol-2-yl, Benzothiazol-2-yl und Chinolin-3-yl, und unsubstituiert oder durch Halogen, Methyl, Pyridyl oder Thienyl monosubstituiert ist;
(6) Eine Verbindung der Formel I, worin R₁ Wasserstoff ist, R₂ Wasserstoff ist, X NH ist und der Rest A unsubstituiertes Pyrid-2-yl oder unsubstituiertes Thiazol-2-yl ist.

Besonders bevorzugt sind im Rahmen der Erfindung die in den Beispielen H1 und H2 genannten Verbindungen der Formel I.

Die vor- und nachstehend verwendeten Allgemeinbegriffe haben, sofern nicht abweichend definiert, die nachfolgend aufgeführten Bedeutungen.

Bei den als Substituenten in Betracht kommenden Halogenatomen handelt es sich sowohl um Fluor und Chlor als auch um Brom und Iod, wobei Fluor, Chlor und Brom bevorzugt und Fluor und Chlor besonders bevorzugt sind Halogen ist dabei als selbständiger Substituent oder als Teil eines Substituenten zu verstehen, wie in Halogenalkyl oder Halogenalkoxy.

Kohlenstoffhaltige Gruppen und Verbindungen enthalten, sofern nicht abweichend definiert, jeweils vorzugsweise 1 bis und mit 4, insbesondere 1 oder 2, Kohlenstoffatome.

Alkyl - als Gruppe per se sowie als Strukturelement von anderen Gruppen und Verbindungen, wie von Alkoxy, Dialkylamino, Halogenalkyl, Halogenalkoxy und Alkoxycarbonyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig oder verzweigt. Als Beispiele für Alkyl seien Methyl, Aethyl, Propyl, Isopropyl, Butyl, Isobutyl, sek.-Butyl und tert.-Butyl sowie Pentyl, Hexyl und jeweils deren Isomere genannt.

Halogensubstituierte Gruppen, d. h. Halogenalkyl und Halogenalkoxy, können teilweise halogeniert oder perhalogeniert sein. Beispiele für Halogenalkyl - als Gruppe per se sowie als Strukturelement von anderen Gruppen und Verbindungen, wie von Halogenalkoxy, sind das ein- bis dreifach durch Fluor, Chlor und/oder Brom substituierte Methyl, wie CHF₂ oder CF₃; das ein- bis fünffach durch Fluor, Chlor und/oder Brom substituierte Aethyl, wie CH₂CF₃, CF₂CF₃, CF₂CCl₃, CF₂CHCl₂, CF₂CHF₂, CF₂CFCl₂, CF₂CHBr₂, CF₂CHClF, CF₂CHBrF oder CClFCHClF; das ein- bis siebenfach durch Fluor, Chlor und/oder Brom substituierte Propyl oder Isopropyl, wie CH₂CHBrCH₂Br, CF₂CHFCF₃, CH₂CF₂CF₃ oder CH(CF₃)₂; und das ein- bis neunfach durch Fluor, Chlor und/oder Brom substituierte Butyl oder eines seiner Isomeren, wie CF(CF₃)CHFCF₃ oder CH₂(CF₂)₂CF₃.

In Dialkylamino können die beiden Alkylgruppen gleich oder verschieden sein.

Als weiterer Gegenstand der Erfindung ist das Verfahren zur Herstellung der Verbindungen der Formel I, in freier Form oder in Salzform, beispielsweise dadurch gekennzeichnet, dass man

### a) eine Verbindung der Formel

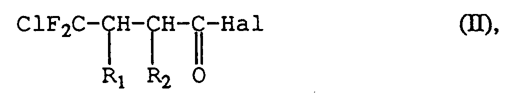

worin R₁ und R₂ die für die Formel I angegebenen Bedeutungen haben und Hal für Halogen, vorzugsweise Chlor oder Brom, steht, in Gegenwart einer Base mit einer Verbindung der Formel

HX-A (III),

worin A und X die für die Formel I angegebenen Bedeutungen haben, oder einem Salz davon umsetzt oder

### b) eine Verbindung der Formel

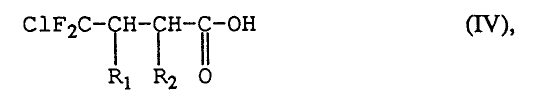

worin R₁ und R₂ die für die Formel I angegebenen Bedeutungen haben, in Gegenwart eines Kondensationsmittels mit einer Verbindung der Formel III oder einem Salz davon umsetzt oder

### c) eine Verbindung der Formel

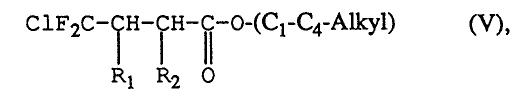

worin R₁ und R₂ die für die Formel I angegebenen Bedeutungen haben, mit einer Verbindung der Formel III oder einem Salz davon umsetzt
und jeweils, wenn erwünscht, eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

Geeignete Basen für die Verfahrensvariante a) sind z. B. organische Basen, beispielsweise Pyridin, 4-Dimethylaminopyridin, Lutidin, Collidin, Trialkylamine, N,N-Dialkylanilin oder bicyclische nicht-nucleophile Basen, wie 1,4-Diazabicyclo[2.2.2]octan (DABCO), 1,5-Diazabicyclo[4.3.0]non-5-en (DBN) und 1,8-Diazabicyclo[5.4.0]undec-7-en (DBU). Die Reaktion wird im allgemeinen bei Temperaturen von -30 bis +70°C, vorzugsweise von -10 bis +50°C, durchgefürt. Man arbeitet dabei zweckmässigerweise in Gegenwart eines inerten Lösungsmittels oder Lösungsmittelgemisches. Es eignen sich hierfür beispielsweise aliphatische und aromatische Kohlenwasserstoffe, wie Benzol, Toluol, Xylole, Petroläther oder Hexan; halogenierte Kohlenwasserstoffe, wie Chlorbenzol, Dichlormethan, Aethylenchlorid, Trichlormethan, Tetrachlormethan oder Tetrachloräthylen; Aether und ätherartige Verbindungen, wie Dialkyläther (Diäthyläther, Diisopropyläther, tert.-Butylmethyläther, etc.), Anisol, Dioxan oder Tetrahydrofuran; Nitrile, wie Acetonitril oder Propionitril; Ester, wie Aethylacetat, Propylacetat oder Butylacetat; Ketone, wie Aceton, Diäthylketon oder Methyläthylketon; und Gemische solcher Lösungsmittel untereinander. Man kann die Reaktion aber auch in einem Ueberschuss einer der oben genannten Basen durchführen oder anstelle der Base auch ein zweites Aequivalent oder auch einen grösseren Ueberschuss der Verbindung III einsetzen.

Geeignete Kondensationsmittel für die Verfahrensvariante b) sind z. B. Phosphorigsäuredichlorid-phenylester, Benzolphosphonsäure-dichlorid, 2,4,6-Trichlor-1,3,5-triazin, 5,6-Dioxo-1,3-diphenyl-5,6-dihydro-〈thieno[3,4-b]-1,4-dioxin〉-2,2-dioxid, Kohlensäurediimidazolid, Dicyclohexylcarbodiimid, Aluminiumoxid, Titan(IV)chlorid, 2,2,4,4,6,6-Hexachlor-1,3,5,2,4,6-triazatriphosphorin und Chlorameisensäureniederalkylester, wie Chlorameisensäureisobutylester. Man arbeitet bevorzugt in Gegenwart einer Base, beispielsweise in Gegenwart eines organischen Amins, z. B. eines Trialkylamins (wie Trimethylamin, Triäthylamin, Tripropylamin oder Diisopropyläthylamin), eines Pyridins (wie Pyridin, 4-Dimethylaminopyridin oder 4-Pyrrolidinopyridin), eines Morpholins (wie N-Methylmorpholin) oder eines N,N-Dialkylanilins (wie N,N-Dimethylanilin oder N-Methyl-N-äthylanilin). Man arbeitet zweckmässigerweise in Gegenwart eines inerten Lösungsmittels oder Lösungsmittelgemisches und bei Temperaturen von -30 bis +70°C, vorzugsweise von -10 bis +50°C. Als Lösungsmittel eignen sich beispielsweise aliphatische und aromatische Kohlenwasserstoffe, wie Benzol, Toluol, Xylole, Petroläther oder Hexan; halogenierte Kohlenwasserstoffe, wie Chlorbenzol, Dichlormethan, Aethylenchlorid, Trichlormethan, Tetrachlormethan oder Tetrachloräthylen; Aether und ätherartige Verbindungen, wie Dialkyläther (Diäthyläther, Diisopropyläther, tert.-Butylmethyläther etc.), Anisol, Dioxan oder Tetrahydrofuran; Nitrile, wie Acetonitril oder Propionitril; Ester, wie Aethylacetat, Propylacetat oder Butylacetat; und Gemische solcher Lösungsmittel untereinander.

Bei der Verfahrensvariante c) werden die Reaktanden mit Vorteil in einem inerten Lösungsmittel oder Lösungsmittelgemisch umgesetzt. Es eignen sich hierfür beispielsweise aliphatische und aromatische Kohlenwasserstoffe, wie Benzol, Toluol, Xylole, Petroläther oder Hexan; halogenierte Kohlenwasserstoffe, wie Chlorbenzol, Dichlormethan, Aethylenchlorid, Trichlormethan, Tetrachlormethan oder Tetrachloräthylen; Aether und ätherartige Verbindungen, wie Dialkyläther (Diäthyläther, Diisopropyläther, tert.-Butylmethyläther etc.), Anisol, Dioxan oder Tetrahydrofuran; Nitrile, wie Acetonitril oder Propionitril; Alkohole, wie Methanol, Aethanol, Propanol oder Isopropanol; und Wasser. Die Aminkomponente III wird mit Vorteil im Ueberschuss verwendet. Die Reaktionstemperaturen liegen in der Regel zwischen 0 und +120°C.

Die Umwandlung von freien Verbindungen I in Salze und von Salzen in freie Verbindungen I oder in andere Salze erfolgt in üblicher Weise, z. B. durch Behandlung einer freien Verbindung I mit einer Säure oder eines Salzes mit einer Base.

Die Verbindungen II, III, IV und V sind bekannt oder können in Analogie zu den bekannten Verbindungen hergestellt werden.

Die erfindungsgemässen Verbindungen I sind bei günstiger Warmblüter-, Fisch- und Pflanzenverträglichkeit wertvolle Wirkstoffe auf dem Gebiet der Schädlingsbekämpfung. Insbesondere wirken die erfindungsgemässen Wirkstoffe gegen Insekten und Spinnentiere, wie sie an Nutz- und Zierpflanzen in der Landwirtschaft und im Gartenbau, insbesondere in Reis-, Baumwoll-, Gemüse- und Obstpflanzungen, und im Forst vorkommen. Die Verbindungen I eignen sich besonders zw Bekämpfung von Insekten in Reis-, Obst- und Gemüsekulturen, insbesondere von pflanzenschädigenden saugenden Insekten, wie von Blattläusen und Zikaden, z. B. von Aphis craccivora, Nilaparvata lugens und Nephotettix cincticeps. Weitere Anwendungsgebiete der erfindungsgemässen Wirkstoffe sind der Vorrats-und Materialschutz sowie im Hygienesektor insbesondere der Schutz von Haus-und Nutztieren. Die Verbindungen I sind gegen alle oder einzelne Entwicklungsstadien von normal sensiblen, aber auch von resistenten Arten von Schädlingen wirksam. Dabei kann sich ihre Wirkung z. B. in einer Abtötung der Schädlinge, welche unmittelbar oder erst nach einiger Zeit, beispielsweise bei einer Häutung, eintritt, oder in einer verminderten Eiablage und/oder Schlupfrate zeigen.

Zu den oben erwähnten Schädlingen gehören:
aus der Ordnung Lepidoptera zum Beispiel
Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prags spp., Scirphaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung Orthoptera zum Beispiel
Blatta spp., Blattella spp., Gryllotalpa spp., Leucophaea maderae, Locusta spp., Periplaneta spp. und Schistocerca spp.;
aus der Ordnung Isoptera zum Beispiel
Reticulitermes spp.;
aus der Ordnung Psocoptera zum Beispiel
Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp. Eurygaster spp.
Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung Hymenoptera zum Beispiel Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis;
aus der Ordnung Thysanura zum Beispiel
Lepisma saccharina und
aus der Ordnung Acarina zum Beispiel
Acarus siro, Aceria sheldoni, Aculus schlechtendali, Amblyomma spp., Argas spp., Boophilus spp., Brevipalpus spp., Bryobia praetiosa Calipitrimerus spp., Chorioptes spp., Dermanyssus gallinae, Eotetranychus carpini, Eriophyes spp., Hyalomma spp., Ixodes spp., Olygonychus pratensis, Ornithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp..

Die gute pestizide Wirkung der erfindungsgemässen Verbindungen I entspricht einer Abtötungsrate (Mortalität) von mindestens 50-60 % der erwähnten Schädlinge.

Die Wirkung der erfindungsgemässen Verbindungen I und der sie enthaltenden Mittel lässt sich durch Zusatz von anderen Insektiziden und/oder Akariziden wesentlich verbreitem und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen I werden in unveränderter Form oder vorzugsweise zusammen mit den in der Formulierungstechnik üblichen Hilfsmitteln eingesetzt und können daher beispielsweise zu emulgierbaren Konzentraten, direkt versprüh- oder verdünnbaren Lösungen, verdünnten Emulsionen, Spritzpulvem, löslichen Pulvern, Stäubemitteln und Granulaten, auch zu Verkapselungen in polymeren Stoffen, in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt. Ferner eignen sich die Verbindungen I auch für den Einsatz bei der Behandlung von Saatgut. Dabei kann sowohl das Saatgut vor dem Säen mit dem Wirkstoff oder einer den Wirkstoff enthaltenden Formulierung behandelt oder gebeizt werden als auch der Wirkstoff beim Säen in die Saatfurche appliziert werden.

Die Formulierungen, das heisst die den Wirkstoff der Formel I, beziehungsweise eine Kombination dieses Wirkstoffs mit anderen Insektiziden und/oder Akariziden, und gegebenenfalls feste oder flüssige Hilfsstoffe enthaltenden Mittel, Zubereitungen oder Zusammensetzungen, werden in bekannter Weise hergestellt, zum Beispiel durch inniges Vermischen und/oder Vermahlen des Wirkstoffs mit den Hilfsstoffen, wie Streckmitteln, z. B. Lösungsmitteln oder festen Trägerstoffen, oder wie oberflächenaktiven Verbindungen (Tensiden).

Als Lösungsmittel können in Frage kommen: Aromatische Kohlenwasserstoffe, bevorzugt die Fraktionen C₈ bis C₁₂ von Alkylbenzolen, wie Xylolgemische oder alkylierte Naphthaline, aliphatische oder cycloaliphatische Kohlenwasserstoffe, wie Cyclohexan, Paraffine oder Tetrahydronaphthalin, Alkohole wie Aethanol, Propanol oder Butanol, Glykole sowie deren Aether und Ester, wie Propylenglykol, Dipropylenglykoläther, Aethylenglykol, Aethylenglykolmonomethyl- oder -äthyläther, Ketone, wie Cyclohexanon, Isophoron oder Diacetanolalkohol, stark polare Lösungsmittel, wie N-Methyl-2-pyrrolidon, Dimethylsulfoxid oder N,N-Dimethylformamid, Wasser sowie gegebenenfalls epoxidierte Pflanzenöle, wie gegebenenfalls epoxidiertes Raps-, Rizinus-, Kokosnuss- oder Sojaöl; gegebenenfalls auch Silikonöle.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, insbesondere Dolomit oder zerkleinerte Pflanzenrückstände, verwendet werden

Als oberflächenaktive Verbindungen kommen je nach Art des zu formulierenden Wirkstoffs I oder der Kombination dieses Wirkstoffs mit anderen Insektiziden und/oder Akariziden nichtionische, kationische und/oder anionische Tenside mit guten Emulgier-, Dispergier- und Netzeigenschaften in Betracht. Unter Tensiden sind auch Tensidgemische zu verstehen.

Als nichtionische Tenside kommen in erster Linie Polyglykolätherderivate von aliphatischen oder cycloaliphatischen Alkoholen, gesättïgten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignet sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten. Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat, in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, Beispiele sind das Stearyltrimethylammoniumchlorid oder das Benzyl-di-(2-chloräthyl)-äthylammoniumbromid.

Geeignete anionische Tenside können sowohl sogenannte wasserlösliche Seifen als auch wasserlösliche synthetische oberflächenaktive Verbindungen sein. Als Seifen eignen sich die Alkali-, Erdalkali- oder gegebenenfalls substituierten Ammoniumsalze von höheren Fettsäuren (C₁₀-C₂₂), wie die Natrium- oder Kalium-Salze der Oel- oder Stearinsäure oder von natürlichen Fettsäuregemischen, die beispielsweise aus Kokosnuss- oder Tallöl gewonnen werden können; ferner sind auch die Fettsäuremethyl-taurin-salze zu erwähnen. Häufiger werden jedoch sogenannte synthetische Tenside verwendet, insbesondere Fensulfonate, Fettsulfate, sulfonierte Benzimidazolderivate oder Alkylarylsulfonate. Die Fettsulfonate oder -sulfate liegen in der Regel als Alkali-, Erdalkali- oder gegebenenfalls substituierte Ammoniumsalze vor und weisen im allgemeinen einen Alkyrest mit 8 bis 22 C-Atomen auf, wobei Alkyl auch den Alkylteil von Acylresten einschliesst; beispielhaft genannt seien das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triäthanolammoniumsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie Salze des Phosphorsäureesters eines p-Nonylphenol-(4-14)-Aethylenoxid-Adduktes oder Phospholipide, in Frage.

Die vorstehend aufgeführten Tenside sind nur als Beispiele anzusehen; in der einschlägigen Literatur werden viele weitere in der Formulierungstechnik gebräuchliche und erfindungsgemäss geeignete Tenside beschrieben.

Die pestiziden Zubereitungen enthalten in der Regel 0,1 bis 99%, insbesondere 0,1 bis 95%, an Wirkstoff I oder an der Kombination dieses Wirkstoffs mit anderen Insektiziden und/oder Akariziden und 1 bis 99,9%, insbesondere 5 bis 99,9%, eines festen oder flüssigen Hilfsstoffes, wobei in der Regel 0 bis 25%, insbesondere 0,1 bis 20%, der Zubereitungen Tenside sein können (% bedeutet jeweils Gewichtsprozent). Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha. Insbesondere setzen sich bevorzugte Formulierungen folgendermassen zusammen (% = Gewichtsprozent):

| Emulgierbare Konzentrate: | |
|---|---|
| Wirkstoff: | 1 bis 90 %, bevorzugt 5 bis 20 % |
| Tensid: | 1 bis 30 %, vorzugsweise 10 bis 20 % |
| flüssiger Trägerstoff: | 5 bis 94 %, vorzugsweise 70 bis 85 % |

| Stäube: | |
|---|---|
| Wirkstoff: | 0,1 bis 10 %, vorzugsweise 0,1 bis 1 % |
| fester Trägerstoff: | 99,9 bis 90 %, vorzugsweise 99,9 bis 99 % |

| Suspensions-Konzentrate: | |
|---|---|
| Wirkstoff: | 5 bis 75 %, vorzugsweise 10 bis 50 % |
| Wasser: | 94 bis 24 %, vorzugsweise 88 bis 30 % |
| Tensid: | 1 bis 40 %, vorzugsweise 2 bis 30 % |

| Benetzbare Pulver: | |
|---|---|
| Wirkstoff: | 0,5 bis 90 %, vorzugsweise 1 bis 80 % |
| Tensid: | 0,5 bis 20 %, vorzugsweise 1 bis 15 % |
| fester Trägerstoff: | 5 bis 95 %, vorzugsweise 15 bis 90 % |

| Granulate: | |
|---|---|
| Wirkstoff: | 0,5 bis 30 %, vorzugsweise 3 bis 15 % |
| fester Trägerstoff: | 99,5 bis 70 %, vorzugsweise 97 bis 85 % |

Die Zubereitungen können auch weitere Hilfsstoffe, wie Stabilisatoren, z. B. gegebenenfalls epoxidierte Pflanzenöle (z. B. epoxidiertes Kokosnussöl, Rapsöl oder Sojaöl), Entschäumer, z. B. Silikonöl, Konservierungsmittel, Viskositätsregulatoren, Bindemittel und/oder Haftmittel, sowie Dünger oder andere Wirkstoffe zur Erzielung spezieller Effekte enthalten

Die folgenden Beispiele dienen der Erläuterung der Erfindung. Sie schränken die Erfindung nicht ein. Temperaturen sind in Grad Celsius angegeben.

### Herstellungsbeispiele

### Beispiel H1:

4-Chlor-4,4-difluor-N-pyrid-2-yl-buttersäureamid (Tabelle 1, Verbindung Nr. 1.1).

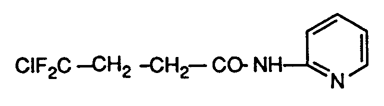

Zu einer Lösung von 2,13 g 2-Aminopyridin und 4,47 g Pyridin in 50 ml Toluol lässt man bei 0° innerhalb von einer halben Stunde 4,0 g 4-Chlor-4,4-difluor-buttersäurechlorid zutropfen. Das Reaktionsgemisch wird 16 Stunden bei Raumtemperatur gerührt, mit 150 ml Diäthyläther verdünnt, nacheinander mit gesättigter NaHCO₃- und gesättigter NaCl-Lösung gewaschen, über MgSO₄ getrocknet und eingedampft. Der Rückstand wird aus Toluol/Hexan umkristallisiert. Man erhält so die Titelverbindung, die bei 64-65° schmilzt.

### Beispiel H2:

In analoger Weise wie in Beispiel H1 beschrieben können auch die anderen in der folgenden Tabelle 1 aufgeführten Verbindungen der Formel I hergestellt werden. In der Spalte "Physik. Daten" dieser Tabelle bezeichnen die angegebenen Temperaturen jeweils den Schmelzpunkt der betreffenden Verbindung.

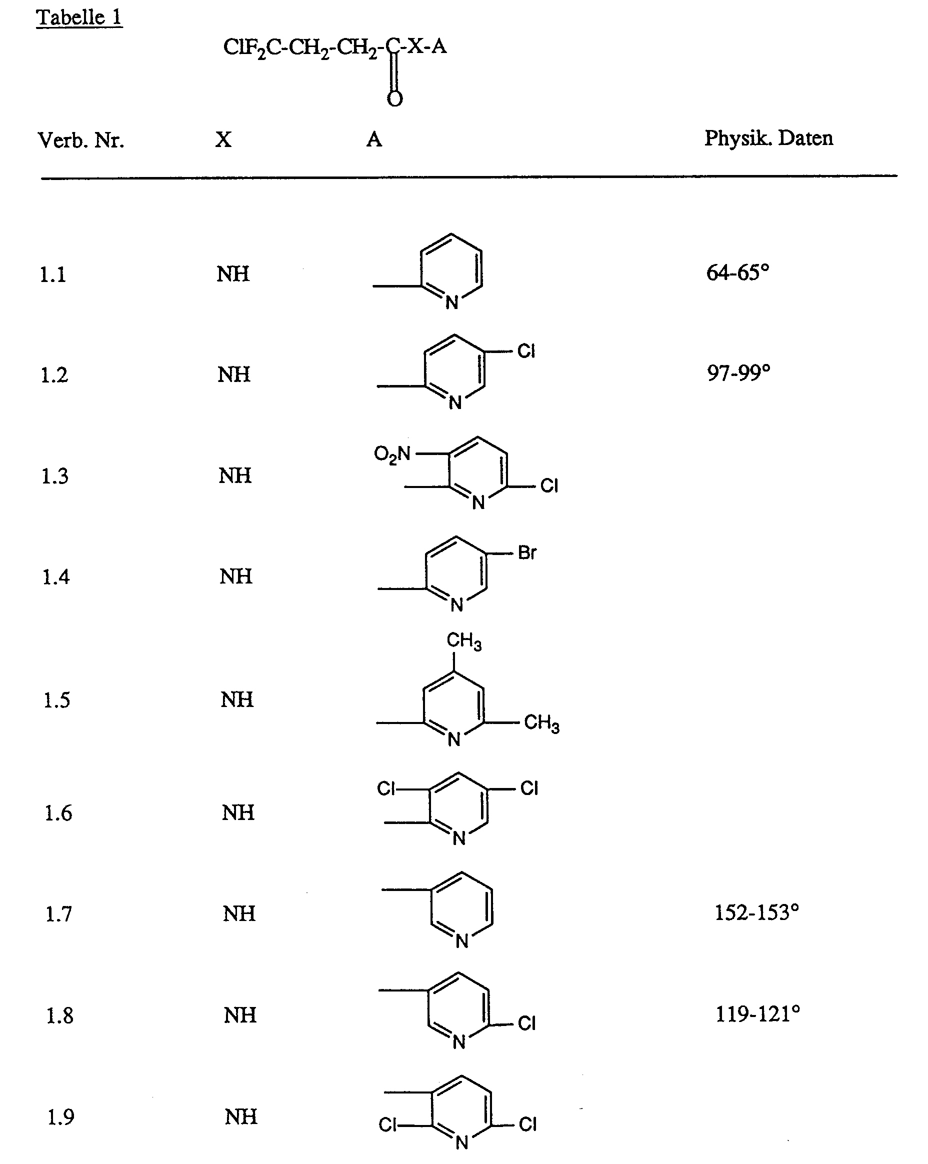

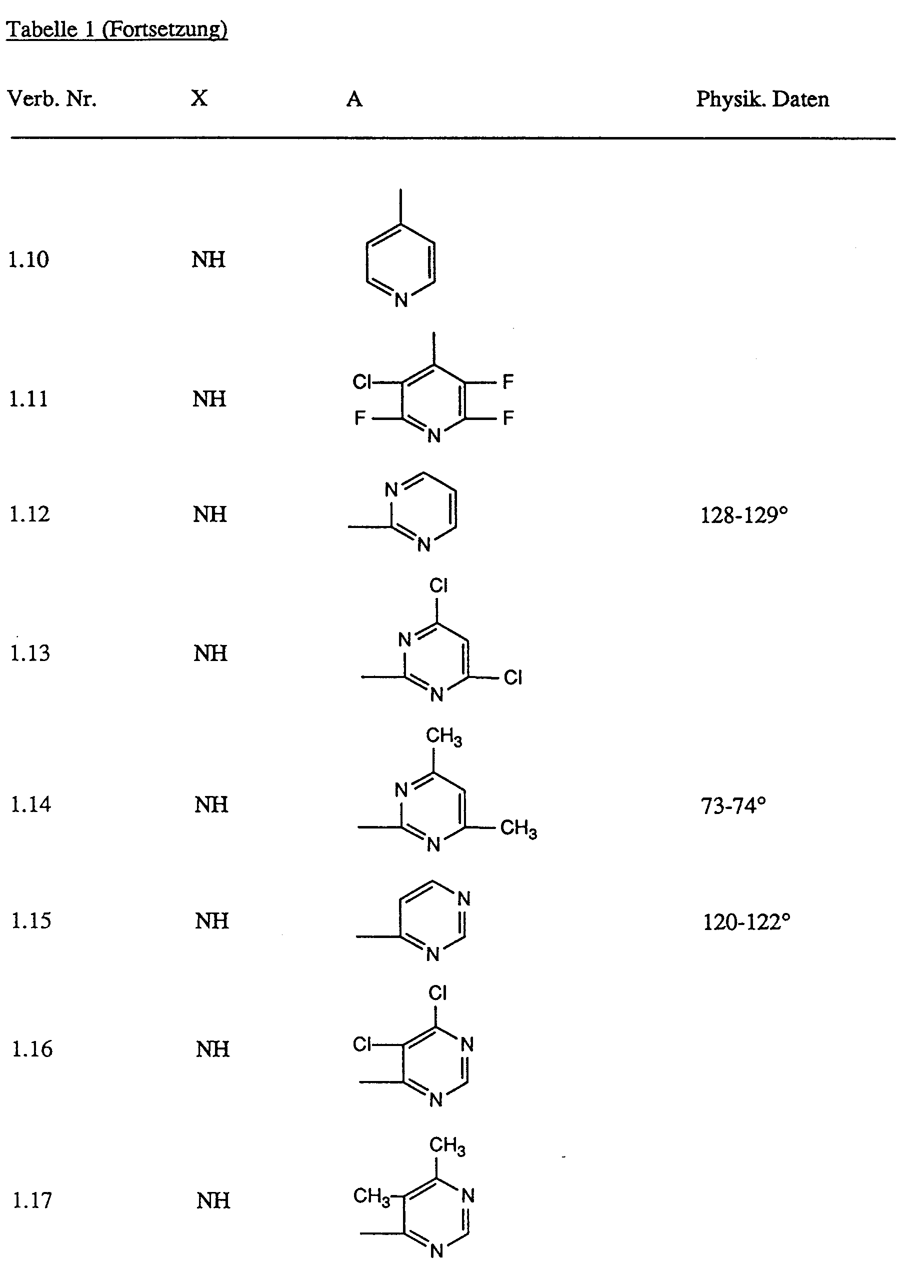

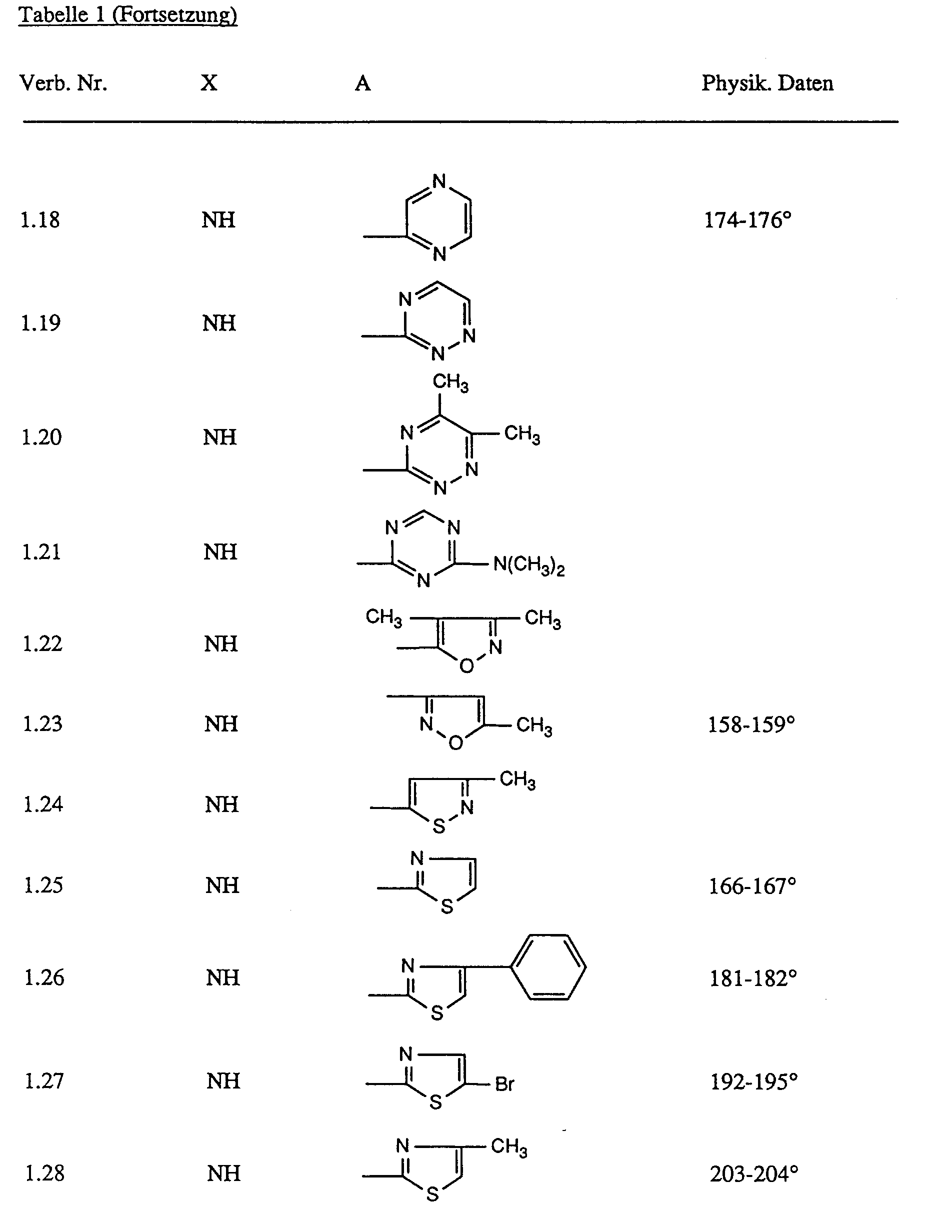

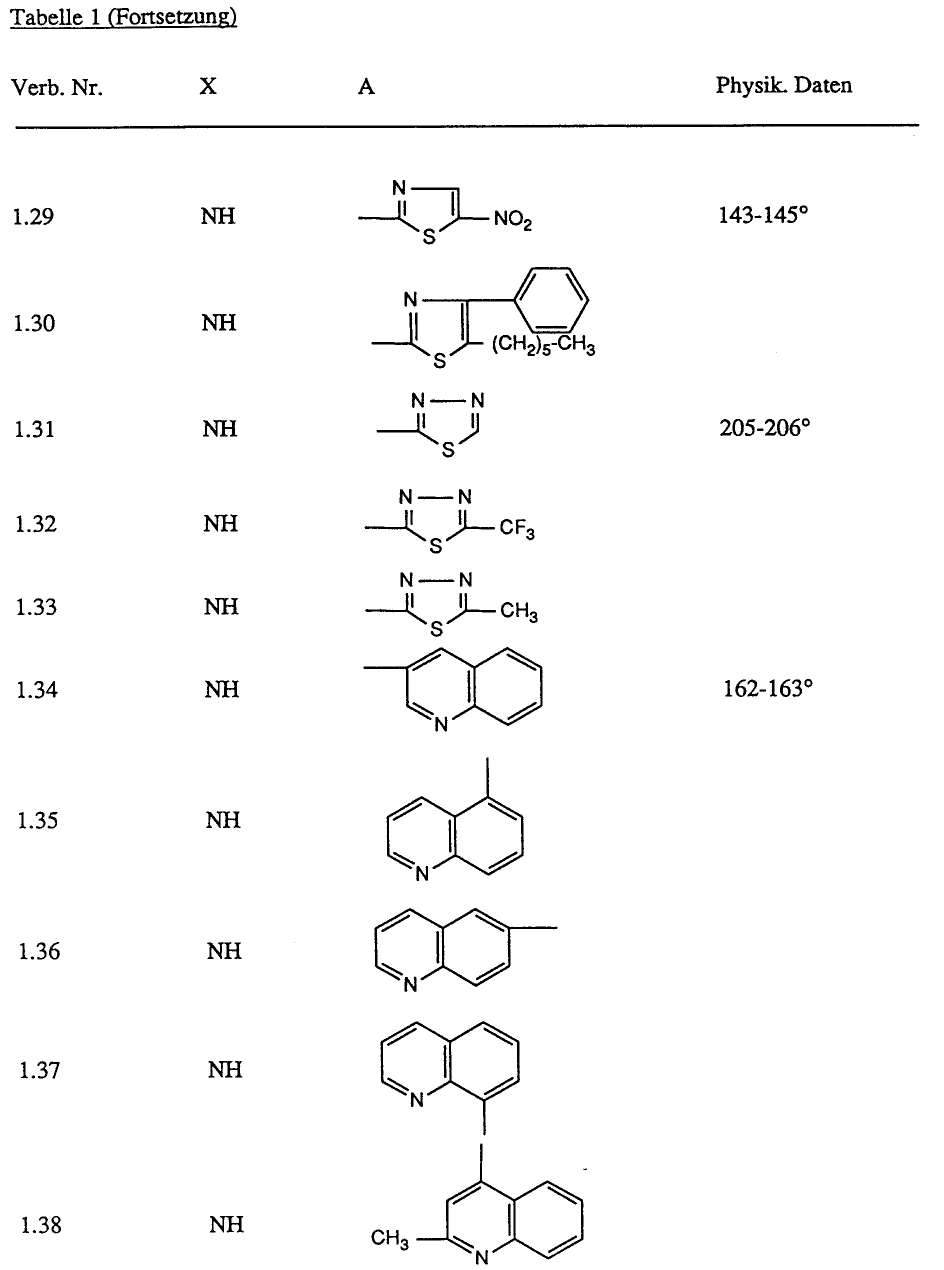

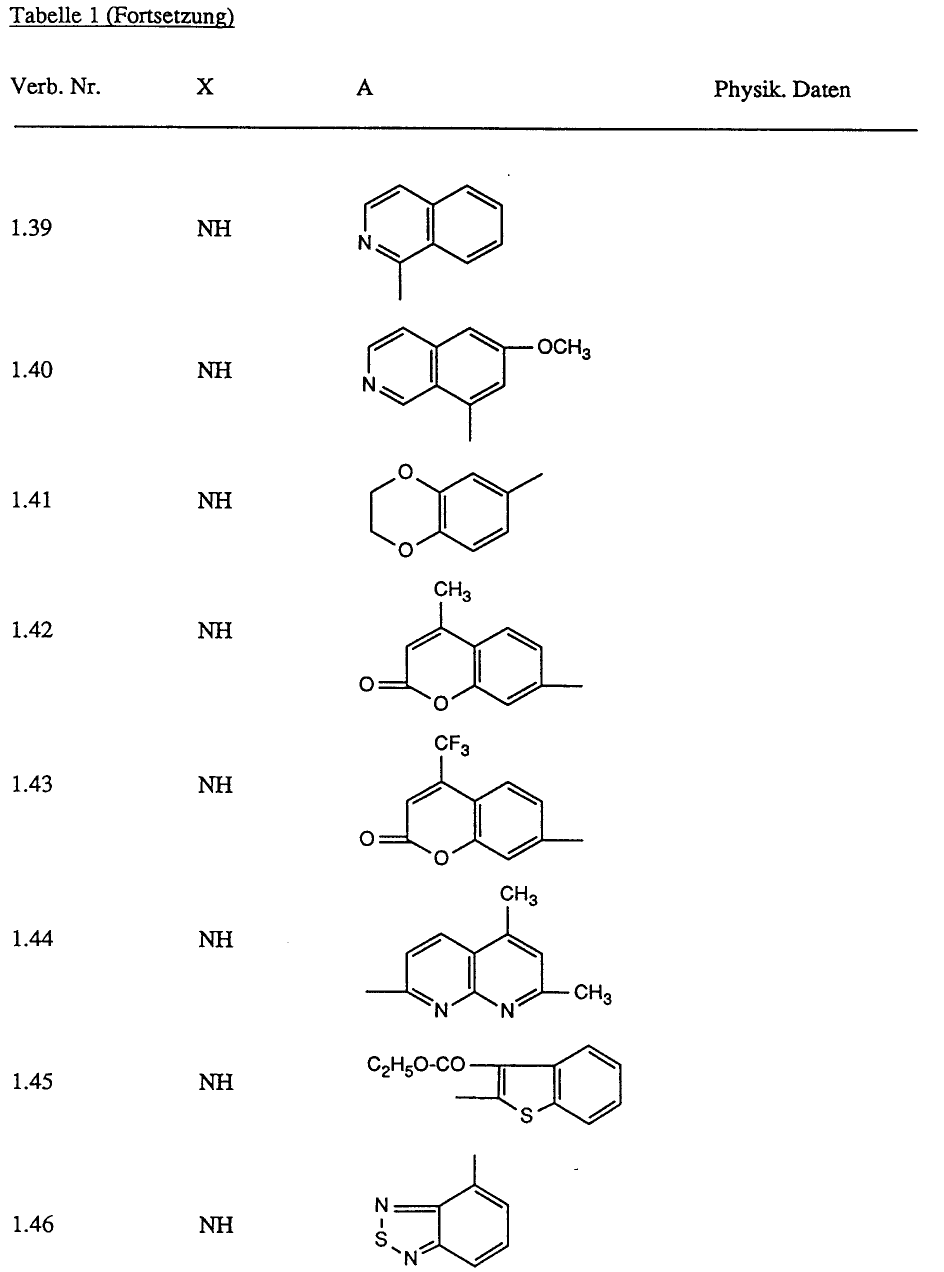

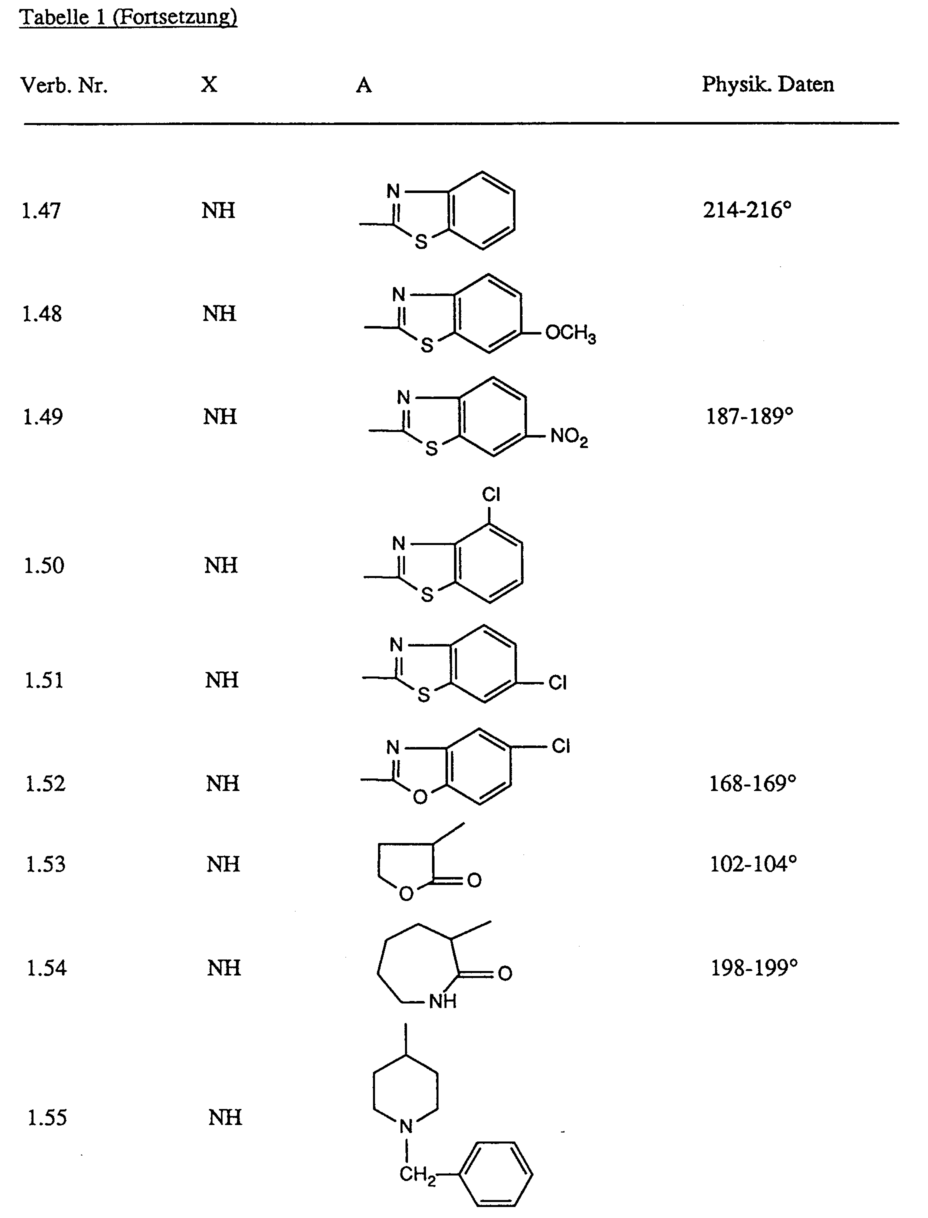

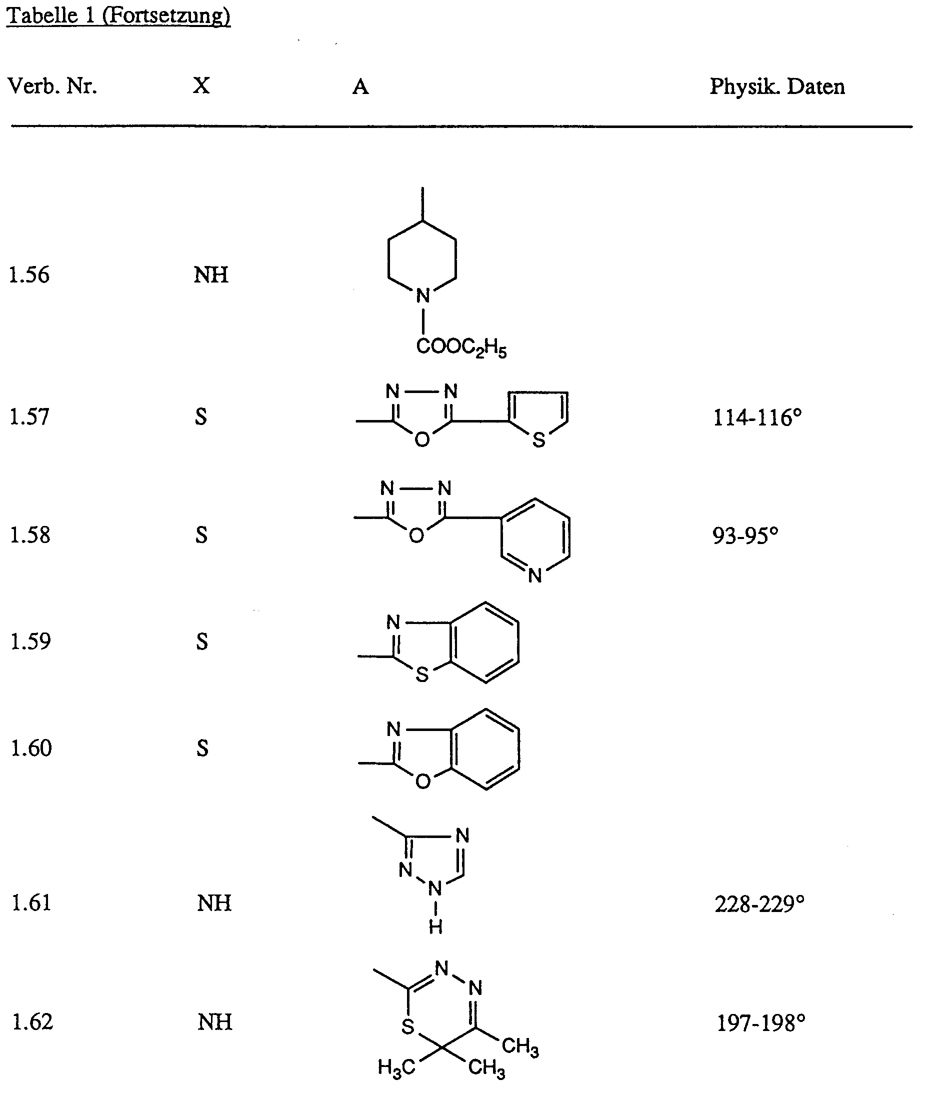

### Formulierungsbeispiele (% = Gewichtsprozent)

| Beispiel F1: Emulsions-Konzentrate | a) | b) | c) |
|---|---|---|---|
| Wirkstoff Nr. 1.1 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff Nr. 1.12 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) | c) | d) |
|---|---|---|---|---|
| Wirkstoff Nr. 1.1 | 5 % | 10 % | 8 % | 21 % |
| Kaolin | 94 % | - | 79 % | 54 % |
| Hochdisperse Kieselsäure | 1 % | - | 13 % | 7 % |
| Attapulgit | - | 90 % | - | 18 % |

Der Wirkstoff wird in Dichlormethan gelöst, auf den Träger aufgesprüht und das Lösungsmittel anschliessend im Vakuum abgedampft.

| Beispiel F4: Stäubemittel | a) | b) |
|---|---|---|
| Wirkstoff Nr. 1.31 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff Nr. 1.1 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 | - | 2 % | - |
| Mol AeO) | | | |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
|---|---|
| Wirkstoff Nr. 1.1 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff Nr. 1.12 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| Beispiel F8: Extruder-Granulat | |
|---|---|
| Wirkstoff Nr. 1.1 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| Beispiel F9: Umhüllungs-Granulat | |
|---|---|
| Wirkstoff Nr. 1.31 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| Beispiel F10: Suspensions-Konzentrat | |
|---|---|
| Wirkstoff Nr. 1.1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

### Biologische Beispiele

### Beispiel B1: Wirkung gegen Boophilus microplus

Vollgesogene adulte Zecken-Weibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung. Verbindungen der Tabelle 1 zeigen in diesem Test gute Wirkung. Insbesondere zeigen die Verbindungen Nr. 1.1, 1.8, 1.12, 1.14, 1.18, 1.25, 1.26, 1.31, 1.47, 1.54, 1.57 und 1.61 eine Wirkung von mehr als 80%.

### Beispiel B2: Wirkung gegen Crocidolmia Binotalis Raupen

Junge Kohlpflanzen werden mit einer wässrigen Emulsions-Spritzbrühe, die 400 ppm des Wirkstoffes enthält, besprüht. Nach dem Antrocknen des Spritzbelages werden die Kohlpflanzen mit 10 Raupen des dritten Stadiums von Crocidolmia binotalis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt. Verbindungen der Tabelle 1 zeigen in diesem Test gute Wirkung. Insbesondere zeigt die Verbindung Nr. 1.25 eine Wirkung von mehr als 80%.

### Beispiel B3: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsions- resp. Suspensions-Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Ueberlebensrate der Wanderlarven der ersten Folgegeneration der behandelten Schildlaus-Population mit derjenigen der unbehandelten Kontrollansätze verglichen.
Verbindungen der Tabelle 1 zeigen in diesem Test gute Wirkung. Insbesondere zeigen die Verbindungen Nr. 1.1, 1.2, 1.12, 1.14, 1.18, 1.23 und 1.58 eine Wirkung von mehr als 80%.

### Beispiel B4: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Verbindungen der Tabelle 1 zeigen in diesem Test gute Wirkung. Insbesondere zeigen die Verbindungen Nr. 1.1, 1.2, 1.7, 1.8, 1.12, 1.14, 1.18, 1.23, 1.25, 1.27, 1.29, 1.31, 1.34, 1.47, 1.52, 1.54, 1.57, 1.58, 1.61 und 1.62 eine Wirkung von mehr als 80%.

### Beispiel B5: Wirkung gegen Tetranychus urticae

Junge Bohnenpflanzen werden mit einer Mischpopulation von Tetranychus urticae besiedelt und 1 Tag später mit einer wässrigen Emulsions-Spritzbrühe, die 400 ppm des Wirkstoffes enthält, besprüht. Die Pflanzen werden anschliessend für 6 Tage bei 25°C inkubiert und danach ausgewertet. Aus dem Vergleich der Anzahl toter Eier, Larven und Adulten auf den behandelten zu denjenigen auf den unbehandelten Pflanzen wird die prozentuale Reduktion der Population (% Wirkung) bestimmt.
Verbindungen der Tabelle 1 zeigen in diesem Test gute Wirkung. Insbesondere zeigen die Verbindungen Nr. 1.1, 1.2, 1.8, 1.12, 1.18, 1.25, 1.34 und 1.53 eine Wirkung von mehr als 80%.

### Beispiel B6: Wirkung gegen Anthonomus grandis Adulte

Junge Baumwollpflanzen werden mit einer wässrigen Emulsions-Spritzbrühe, die 400 ppm des Wirkstoffes enthält, besprüht. Nach dem Antrocknen des Spritzbelages werden die Baumwollpflanzen mit 10 Adulten von Anthonomus grandis besiedelt und in einen Plastikbehälter gegeben. 3 Tage später erfolgt die Auswertung. Aus dem Vergleich der Anzahl toter Käfer und des Frasschadens auf den behandelten zu denjenigen auf den unbehandelten Pflanzen wird die prozentuale Reduktion der Population bezw. die prozentuale Reduktion des Frasschadens (% Wirkung) bestimmt.
Verbindungen der Tabelle 1 zeigen in diesem Test gute Wirkung. Insbesondere zeigen die Verbindungen Nr. 1.1, 1.7, 1.12, 1.23, 1.25, 1.31, 1.52, 1.54 und 1.58 eine Wirkung von mehr als 80%.

### Beispiel B7: Wirkung gegen Aphis craccivora

Erbsenkeimlinge werden mit Aphis craccivora infiziert und anschliessend mit einer Spritzbrühe, die 400 ppm des Wirkstoffes enthält, besprüht und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.
Verbindungen der Tabelle 1 zeigen in diesem Test gute Wirkung. Insbesondere zeigen die Verbindungen Nr. 1.1, 1.7, 1.8, 1.12, 1.15, 1.23, 1.57, 1.58 und 1.62 eine Wirkung von mehr als 80%.

### Beispiel B8: Systemische Wirkung gegen Myzus persicae

Erbsenkeimlinge werden mit Myzus persicae infiziert, anschliessend mit den Wurzeln in eine Spritzbrühe, die 400 ppm des Wirkstoffes enthält, gestellt und bei 20°C inkubiert. 3 und 6 Tage später erfolgt die Auswertung. Aus dem Vergleich der Anzahl toter Blattläuse auf den behandelten zu denjenigen auf den unbehandelten Pflanzen wird die prozentuale Reduktion der Population (% Wirkung) bestimmt.
Verbindungen der Tabelle 1 zeigen in diesem Test gute Wirkung. Insbesondere zeigen die Verbindungen Nr. 1.1, 1.2, 1.12, 1.15 und 1.23 eine Wirkung von mehr als 80%.

### Beispiel B9: Systemische Wirkung gegen Nilaparvata lugens

Töpfe mit Reispflanzen werden in eine wässrige EmulsionsLösung, die 400 ppm des Wirkstoffes enthält, gestellt. Anschliessend werden die Reispflanzen mit larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.
Verbindungen der Tabelle 1 zeigen in diesem Test gute Wirkung. Insbesondere zeigen die Verbindungen Nr. 1.1, 1.2, 1.7, 1.8, 1.12, 1.14, 1.15, 1.18, 1.23, 1.25, 1.26, 1.27, 1.28, 1.29, 1.31, 1.34, 1.47, 1.49, 1.52, 1.53, 1.54, 1.57, 1.58, 1.61 und 1.62 eine Wirkung von mehr als 80%.

### Beispiel B10: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensraten der Raupen im Vergleich zu unbehandelten Kontrollansätzen ausgewertet (% Reduktion der Population).
Verbindungen der Tabelle 1 zeigen in diesem Test gute Wirkung. Insbesondere zeigen die Verbindungen Nr. 1.1, 1.2, 1.7, 1.8, 1.12, 1.15, 1.18, 1.23, 1.25, 1.26, 1.27, 1.31, 1.45, 1.47, 1.49, 1.52, 1.57, 1.58 und 1.62 eine Wirkung von mehr als 80%.

### Beispiel B11: Systemische Wirkung gegen Nephotettix cincticeps

Töpfe mit Reispflanzen werden in eine wässrige Emulsions-Lösung, die 400 ppm des Wirkstoffes enthält, gestellt. Anschliessend werden die Reispflanzen mit Larven des 2. und 3. Stadiums besiedelt. 6 Tage später erfolgt die Auswertung. Aus dem Vergleich der Anzahl Zikaden auf den behandelten zu denjenigen auf den unbehandelten Pflanzen wird die prozentuale Reduktion der Population (% Wirkung) bestimmt.
Verbindungen der Tabelle 1 zeigen in diesem Test gute Wirkung. Insbesondere zeigen die Verbindungen Nr. 1.7, 1.18, 1.23, 1.25 und 1.58 eine Wirkung von mehr als 80%.

### Beispiel B12: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf dem behandelten zu denjenigen auf den unbehandelten Pflanzen wird die prozentuale Reduktion der Population bzw. die prozentuale Reduktion des Frassschadens (% Wirkung) bestimmt.
Verbindungen der Tabelle 1 zeigen in diesem Test gute Wirkung. Insbesondere zeigen die Verbindungen Nr. 1.1, 1.7, 1.12, 1.23 und 1.58 eine Wirkung von mehr als 80%.

### Beispiel B13: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit Zikadenlarven des 2. und 3. Stadiums besiedelt. 21 Tage später erfolgt die Auswertung. Aus dem Vergleich der Anzahl überlebender Zikaden auf den behandelten zu denjenigen auf den unbehandelten Pflanzen wird die prozentuale Reduktion der Population (% Wirkung) bestimmt.
Verbindungen der Tabelle 1 zeigen in diesem Test gute Wirkung. Insbesondere zeigen die Verbindungen Nr. 1.1, 1.18, 1.23, 1.25, 1.29, 1.31, 1.34, 1.52, 1.58 und 1.62 eine Wirkung von mehr als 80%.

## Patentansprüche

1. Eine Verbindung der Formel 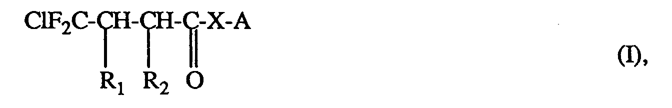 worin
A einen substituierten oder unsubstituierten, über eines seiner Kohlenstoffatome an X gebundenen, aromatischen oder nicht-aromatischen, mono- oder bicyclischen, heterocyclischen Rest;
R₁ und R₂ unabhängig voneinander Wasserstoff oder C₁-C₆-Alkyl;
X NR₃, O oder S; und
R₃ Wasserstoff oder C₁-C₄-Alkyl
bedeuten,
in freier Form oder in Salzform.

2. Eine Verbindung gemäss Anspruch 1 der Formel I in freier Form.

3. Eine Verbindung gemäss Anspruch 2 der Formel I, worin R₁ Wasserstoff ist.

4. Eine Verbindung gemäss Anspruch 2 der Formel I, worin R₂ Wasserstoff ist.

5. Eine Verbindung gemäss Anspruch 2 der Formel I, worin X NR₃ oder S und R₃ Wasserstoff oder C₁-C₄-Alkyl ist.

6. Eine Verbindung gemäss Anspruch 2 der Formel I, worin der, über eines seiner Kohlenstoffatome an X gebundene, Rest A ausgewählt ist aus der Gruppe von Resten, bestehend aus 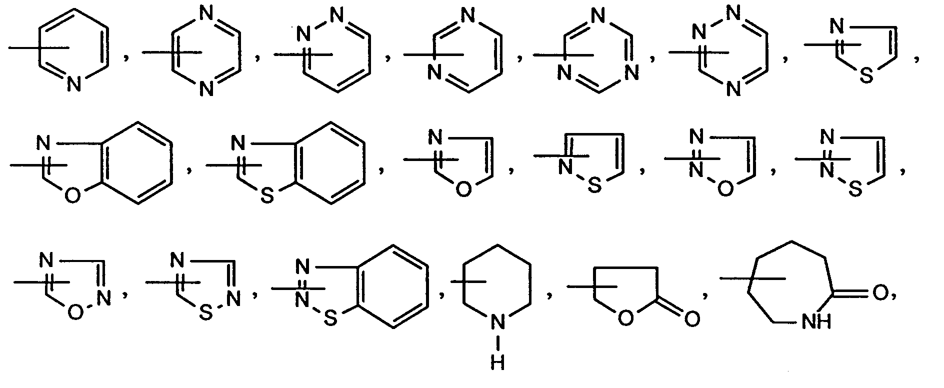 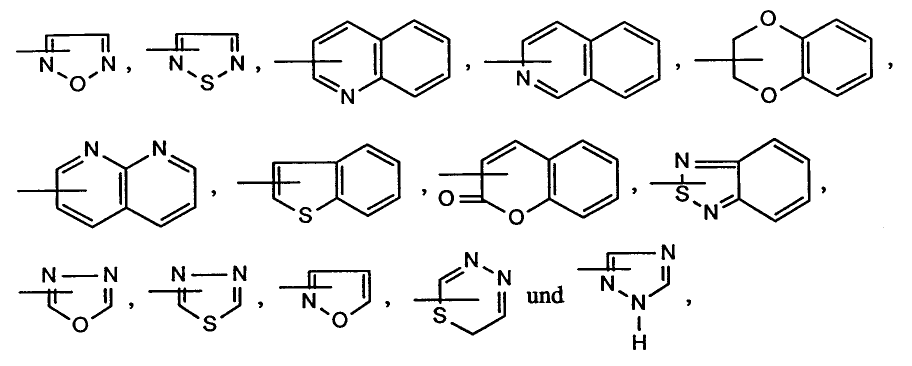 und unsubstituiert ist oder einen, zwei, drei oder vier Substituenten, ausgewählt aus der Gruppe, bestehend aus Halogen, C₁-C₆-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, Nitro, Cyano, C₁-C₄-Alkoxycarbonyl, Di-C₁-C₄-alkylamino, Phenyl, Benzyl, Pyridyl, Thienyl und durch Halogen, C₁-C₄-Alkyl, C₁-C₄-Halogenalkyl mit 1 bis 9 Halogenatomen, C₁-C₄-Alkoxy, Nitro oder Cyano einfach substituiertem Phenyl, Benzyl, Pyridyl oder Thienyl, trägt.

7. Eine Verbindung gemäss Anspruch 6 der Formel I, worin der, über eines seiner Kohlenstoffatome an X gebundene, Rest A ausgewählt ist aus der Gruppe von Resten, bestehend aus 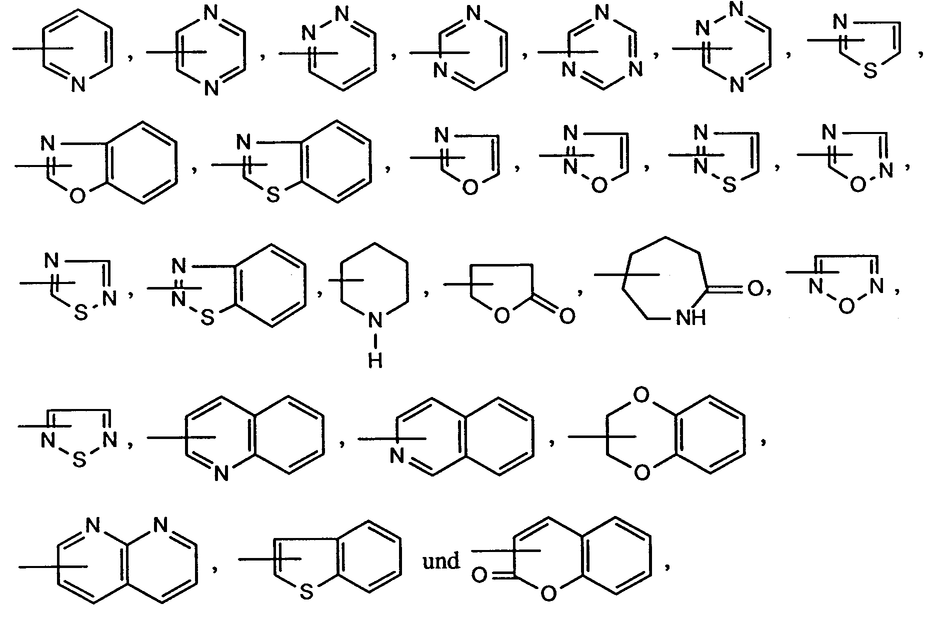 und unsubstituiert ist oder einen oder zwei Substituenten, ausgewählt aus der Gruppe, bestehend aus Halogen, C₁-C₆-Alkyl, C₁-C₄-Halogenalkyl mit 1 bis 9 Halogenatomen, C₁-C₄-Alkoxy, C₁-C₄-Halogenalkoxy mit 1 bis 9 Halogenatomen, Nitro, Cyano, C₁-C₄-Alkoxycarbonyl, Di-C₁-C₄-alkylamino, Phenyl, Benzyl, Pyridyl, Thienyl und durch Halogen, C₁-C₄-Alkyl, C₁-C₄-Halogenalkyl mit 1 bis 9 Halogenatomen, C₁-C₄-Alkoxy, Nitro oder Cyano einfach substituiertem Phenyl, Benzyl, Pyridyl oder Thienyl, trägt.

8. Eine Verbindung gemäss Anspruch 6 der Formel I, worin der, über eines seiner Kohlenstoffatome an X gebundene, Rest A ausgewählt ist aus der Gruppe von Resten, bestehend aus 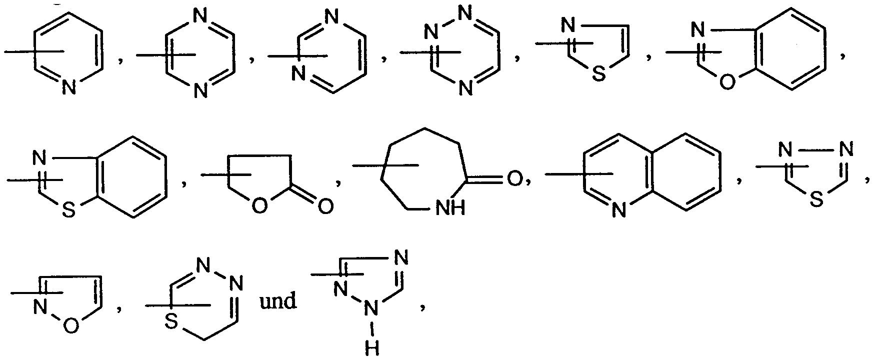 und unsubstituiert ist oder einen, zwei oder drei Substituenten, ausgewählt aus der Gruppe, bestehend aus Halogen, C₁-C₆-Alkyl, Nitro, Phenyl, Pyridyl und Thienyl, trägt.

9. Eine Verbindung gemäss Anspruch 6 der Formel I, worin der Rest A ausgewählt ist aus der Gruppe von Resten, bestehend aus Pyrid-2-yl, Pyrid-3-yl, Pyrimidin-2-yl, Pyrazin-2-yl, Thiazol-2-yl, Isoxazol-3-yl, 1,3,4-Oxadiazol-2-yl, 1,3,4-Thiadiazol-2-yl, Benzoxazol-2-yl, Benzothiazol-2-yl und Chinolin-3-yl, und unsubstituiert oder durch Halogen, Methyl, Pyridyl oder Thienyl monosubstituiert ist.

10. Eine Verbindung gemäss Anspruch 9 der Formel I, worin der Rest A unsubstituiertes Pyrid-2-yl oder unsubstituiertes Thiazol-2-yl ist.

11. Eine Verbindung gemäss Anspruch 9 der Formel I, worin R₁ Wasserstoff ist, R₂ Wasserstoff ist, X NH oder S ist und der Rest A ausgewählt ist aus der Gruppe von Resten, bestehend aus Pyrid-2-yl, Pyrid-3-yl, Pyrimidin-2-yl, Pyrazin-2-yl, Thiazol-2-yl, Isoxazol-3-yl, 1,3,4-Oxadiazol-2-yl, 1,3,4-Thiadiazol-2-yl, Benzoxazol-2-yl, Benzothiazol-2-yl und Chinolin-3-yl, und unsubstituiert oder durch Halogen, Methyl, Pyridyl oder Thienyl monosubstituiert ist.

12. Eine Verbindung gemäss Anspruch 11 der Formel I, worin R₁ Wässerstoff ist, R₂ Wasserstoff ist, X NH ist und der Rest A unsubstituiertes Pyrid-2-yl oder unsubstituiertes Thiazol-2-yl ist.

13. Eine Verbindung gemäss Anspruch 12 der Formel

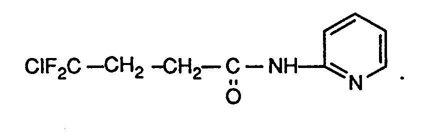

14. Eine Verbindung gemäss Anspruch 11 der Formel

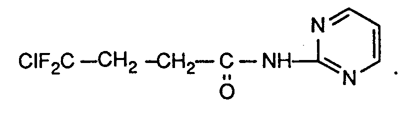

15. Eine Verbindung gemäss Anspruch 11 der Formel

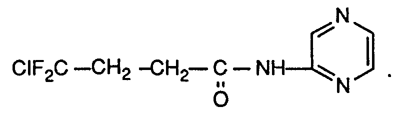

16. Eine Verbindung gemäss Anspruch 11 der Formel

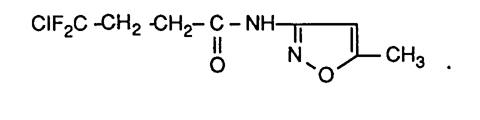

17. Eine Verbindung gemäss Anspruch 11 der Formel

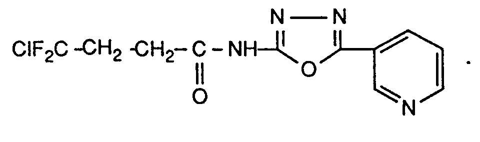

18. Eine Verbindung gemäss Anspruch 12 der Formel

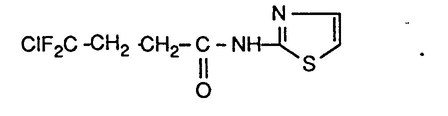

19. Verfahren zur Herstellung einer Verbindung gemäss Anspruch 1 der Formel I, in freier Form oder in Salzform, dadurch gekennzeichnet, dass man
a) eine Verbindung der Formel 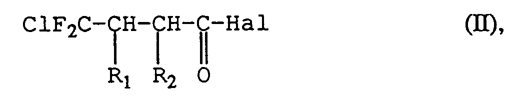 worin R₁ und R₂ die für die Formel I angegebenen Bedeutungen haben und Hal für Halogen, vorzugsweise Chlor oder Brom, steht, in Gegenwart einer Base mit einer Verbindung der Formel
HX-A (III),
worin A und X die für die Formel I angegebenen Bedeutungen haben, oder einem Salz davon umsetzt oder
b) eine Verbindung der Formel 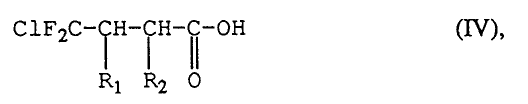 worin R₁ und R₂ die für die Formel I angegebenen Bedeutungen haben, in Gegenwart eines Kondensationsmittels mit einer Verbindung der Formel III oder einem Salz davon umsetzt oder
c) eine Verbindung der Formel 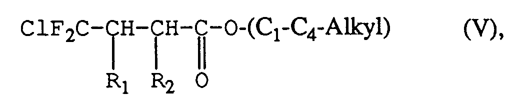 worin R₁ und R₂ die für die Formel I angegebenen Bedeutungen haben, mit einer Verbindung der Formel III oder einem Salz davon umsetzt
und jeweils, wenn erwünscht, eine verfahrensgemäss erhältliche freie Verbindung der Formel I in ein Salz oder ein verfahrensgemäss erhältliches Salz einer Verbindung der Formel I in die freie Verbindung der Formel I oder in ein anderes Salz überführt.

20. Schädlingsbekämpfungsmittel, dadurch gekennzeichnet, dass es mindestens eine Verbindung gemäss Anspruch 1 der Formel I, in freier Form oder in agrochemisch verwendbarer Salzform, als Wirkstoff und mindestens einen Hilfsstoff enthält.

21. Mittel gemäss Anspruch 20 zur Bekämpfung von Insekten und/oder Spinnentieren.

22. Verfahren zur Herstellung eines Mittels gemäss Anspruch 20, dadurch gekennzeichnet, dass man den Wirkstoff mit dem (den) Hilfsstoff(en) innig vermischt.

23. Verwendung einer Verbindung gemäss Anspruch 1 der Formel I, in freier Form oder in agrochemisch verwendbarer Salzform, oder eines Mittels gemäss Anspruch 20 zur Bekämpfung von Schädlingen.

24. Verwendung gemäss Anspruch 23 zur Bekämpfung von Insekten und/oder Spinnentieren.

25. Verwendung gemäss Anspruch 23 zur Behandlung von Saatgut.

26. Verfahren zur Bekämpfung von Schädlingen, dadurch gekennzeichnet, dass man als Wirkstoff eine Verbindung gemäss Anspruch 1 der Formel I, in freier Form oder in agrochemisch verwendbarer Salzform, auf die Schädlinge und/oder ihren lebensraum appliziert.

27. Verfahren gemäss Anspruch 26 zur Bekämpfung von Insekten und/oder Spinnentieren.

28. Verfahren gemäss Anspruch 26 zum Schutz von Saatgut, dadurch gekennzeichnet, dass man das Saatgut oder die Saatfurche behandelt.

29. Saatgut, behandelt gemäss dem in Anspruch 28 beschriebenen Verfahren.

## Claims

1. A compound of formula 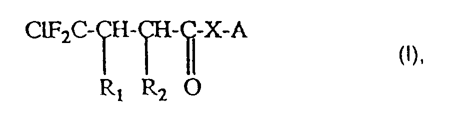 wherein
A is a substituted or unsubstituted, aromatic or non-aromatic, monocyclic or bicyclic heterocyclic radical that is bonded by way of one of its carbon atoms to X; each of R₁ and R₂, independently of the other, is hydrogen or C₁-C₆alkyl;
X is NR₃, O or S; and
R₃ is hydrogen or C₁-C₄alkyl,
in free form or in salt form.

2. A compound according to claim 1 of formula I in free form.

3. A compound according to claim 2 of formula I, wherein R₁ is hydrogen.

4. A compound according to claim 2 of formula I, wherein R₂ is hydrogen.

5. A compound according to claim 2 of formula I, wherein X is NR₃ or S and R₃ is hydrogen or C₁-C₄alkyl.

6. A compound according to claim 2 of formula I, wherein the radical A that is bonded by way of one of its carbon atoms to X is selected from the group of radicals consisting of 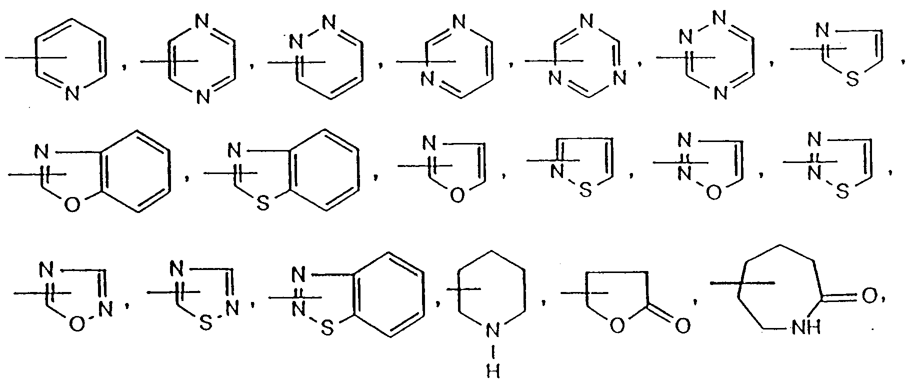 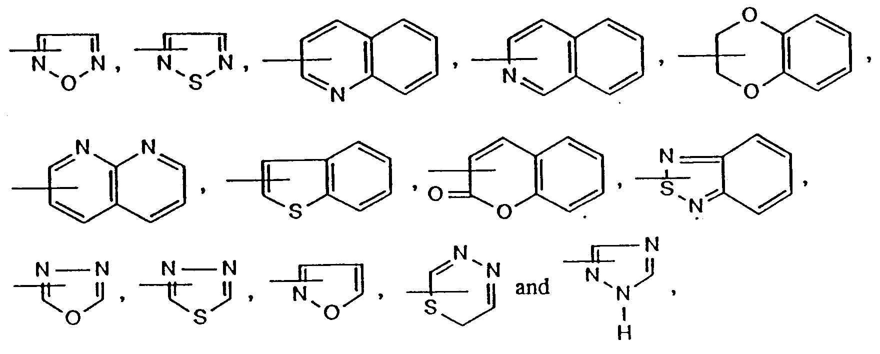 and is unsubstituted or carries one, two, three or four substituents selected from the group consisting of halogen, C₁-C₆alkyl, C₁-C₄haloalkyl having from 1 to 9 halogen atoms, C₁-C₄alkoxy, C₁-C₄haloalkoxy having from 1 to 9 halogen atoms, nitro, cyano, C₁-C₄alkoxycarbonyl, di(C₁-C₄alkyl)amino, phenyl, benzyl, pyridyl, thienyl and phenyl, benzyl, pyridyl and thienyl each mono-substituted by halogen, C₁-C₄alkyl, C₁-C₄haloalkyl having from 1 to 9 halogen atoms, C₁-C₄alkoxy, nitro or by cyano.

7. A compound according to claim 6 of formula I, wherein the radical A that is bonded by way of one of its carbon atoms to X is selected from the group of radicals consisting of 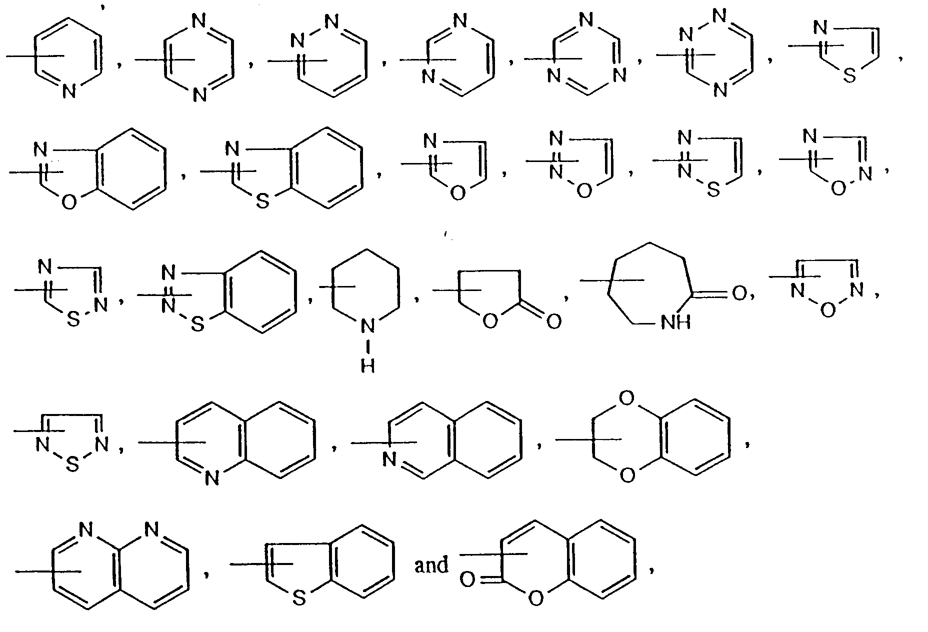 and is unsubstituted or carries one or two substituents selected from the group consisting of halogen, C₁-C₆alkyl, C₁-C₄haloalkyl having from 1 to 9 halogen atoms, C₁-C₄alkoxy, C₁-C₄haloalkoxy having from 1 to 9 halogen atoms, nitro, cyano, C₁-C₄alkoxycarbonyl, di(C₁-C₄alkyl)amino, phenyl, benzyl, pyridyl, thienyl and phenyl, benzyl, pyridyl and thienyl each mono-substituted by halogen, C₁-C₄alkyl, C₁-C₄haloalkyl having from 1 to 9 halogen atoms, C₁-C₄alkoxy, nitro or by cyano.

8. A compound according to claim 6 of formula I, wherein the radical A that is bonded by way of one of its carbon atoms to X is selected from the group of radicals consisting of 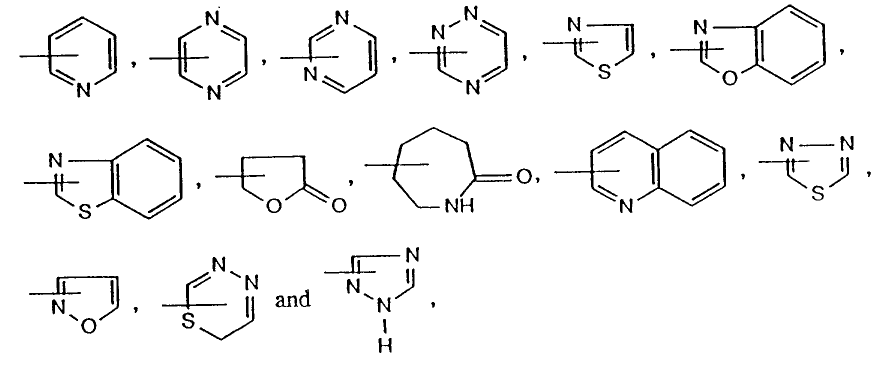 and is unsubstituted or carries one, two or three substituents selected from the group consisting of halogen, C₁-C₆alkyl, nitro, phenyl, pyridyl and thienyl.

9. A compound according to claim 6 of formula I, wherein the radical A is selected from the group of radicals consisting of pyrid-2-yl, pyrid-3-yl, pyrimidin-2-yl, pyrazin-2-yl, thiazol-2-yl, isoxazol-3-yl, 1,3,4-oxadiazol-2-yl, 1,3,4-thiadiazol-2-yl, benzoxazol-2-yl, benzothiazol-2-yl and quinolin-3-yl and is unsubstituted or mono-substituted by halogen, methyl, pyridyl or by thienyl.

10. A compound according to claim 9 of formula I, wherein the radical A is unsubstituted pyrid-2-yl or unsubstituted thiazol-2-yl.

11. A compound according to claim 9 of formula I, wherein R₁ is hydrogen, R₂ is hydrogen, X is NH or S and the radical A is selected from the group of radicals consisting of pyrid-2-yl, pyrid-3-yl, pyrimidin-2-yl, pyrazin-2-yl, thiazol-2-yl, isoxazol-3-yl, 1,3,4-oxadiazol-2-yl, 1,3,4-thiadiazol-2-yl, benzoxazol-2-yl, benzothiazol-2-yl and quinolin-3-yl and is unsubstituted or mono-substituted by halogen, methyl, pyridyl or by thienyl.

12. A compound according to claim 11 of formula I, wherein R₁ is hydrogen, R₂ is hydrogen, X is NH and the radical A is unsubstituted pyrid-2-yl or unsubstituted thiazol-2-yl.

13. A compound according to claim 12 of the formula

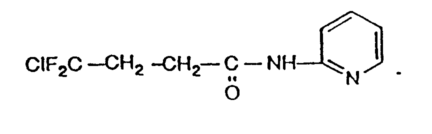

14. A compound according to claim 11 of the formula

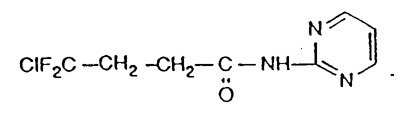

15. A compound according to claim 11 of the formula

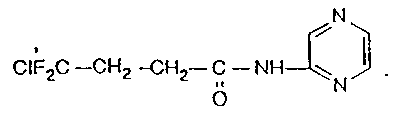

16. A compound according to claim 11 of the formula

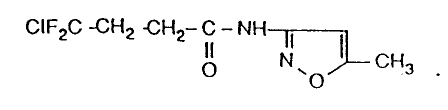

17. A compound according to claim 11 of the formula

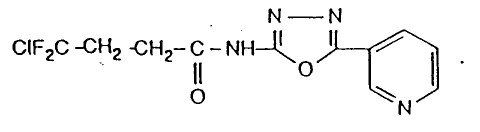

18. A compound according to claim 12 of the formula

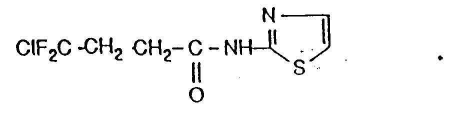

19. A process for the preparation of a compound according to claim 1 of formula I, in free form or in salt form, wherein
a) a compound of formula 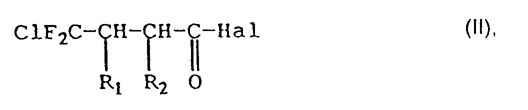 wherein R₁ and R₂ are as defined for formula I and Hal is halogen, preferably chlorine or bromine, is reacted in the presence of a base with a compound of formula
HX-A (III),
wherein A and X are as defined for formula I, or with a salt thereof, or
b) a compound of formula 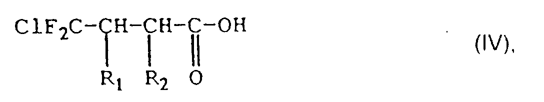 wherein R₁ and R₂ are as defined for formula I, is reacted in the presence of a condensation agent with a compound of formula III, or with a salt thereof, or
c) a compound of formula 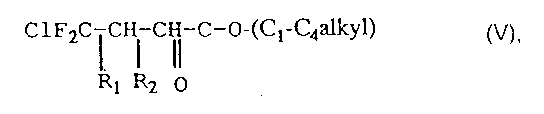 wherein R₁ and R₂ are as defined for formula I, is reacted with a compound of formula III, or with a salt thereof,
and in each case, if desired, a free compound of formula I obtainable in accordance with the process is converted into a salt or a salt of a compound of formula I obtainable in accordance with the process is converted into the free compound of formula I or into a different salt.

20. A pesticidal composition comprising as active ingredient at least one compound according to claim 1 of formula I, in free form or in agrochemically acceptable salt form, and at least one adjuvant.

21. A composition according to claim 20 for controlling insects and/or arachnids.

22. A process for the preparation of a composition according to claim 20, wherein the active ingredient is intimately mixed with the adjuvant(s).

23. The use of a compound according to claim 1 of formula I, in free form or in agrochemically acceptable salt form, or of a composition according to claim 20 for controlling pests.

24. The use according to claim 23 for controlling insects and/or arachnids.

25. The use according to claim 23 for treating seeds.

26. A method of controlling pests, wherein, as active ingredient, a compound according to claim 1 of formula I, in free form or in agrochemically acceptable salt form, is applied to the pests and/or to the habitat thereof.

27. A method according to claim 26 for controlling insects and/or arachnids.

28. A method according to claim 26 for protecting seeds, which comprises treating the seeds or the furrow.

29. Seeds treated in accordance with the method described in claim 28.

## Revendications

1. Un composé de formule 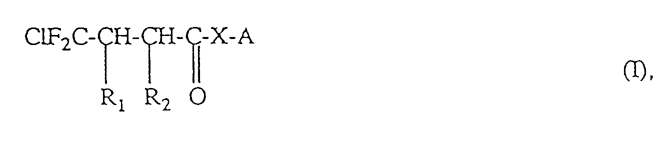 dans laquelle
A représente un radical hétérocyclique mono- ou bicyclique, aromatique ou non aromatique, substitué ou non, relié à X par l'un de ses atomes de carbone ;
R₁ et R₂ représentent chacun, indépendamment l'un de l'autre, l'hydrogène ou un groupe alkyle en C1-C6 ;
X représente Nr₃, O ou S ; et
R₃ représente l'hydrogène ou un groupe alkyle en C1-C4,
à l'état libre ou à l'état de sels.

2. Un composé selon revendication 1, de formule I à l'état libre.

3. Un composé selon revendication 2, de formule I dans laquelle R₁ représente l'hydrogène.

4. Un composé selon revendication 2, de formule I dans laquelle R₂ représente l'hydrogène.

5. Un composé selon revendication 2, répondant à la formule I dans laquelle X représente NR₃ ou S et R₃ représente l'hydrogène ou un groupe alkyle en C1-C4.

6. Un composé selon revendication 2, répondant à la formule I dans laquelle le radical A relié à X par l'intermédiaire de l'un de ses atomes de carbone est choisi parmi les radicaux suivants : 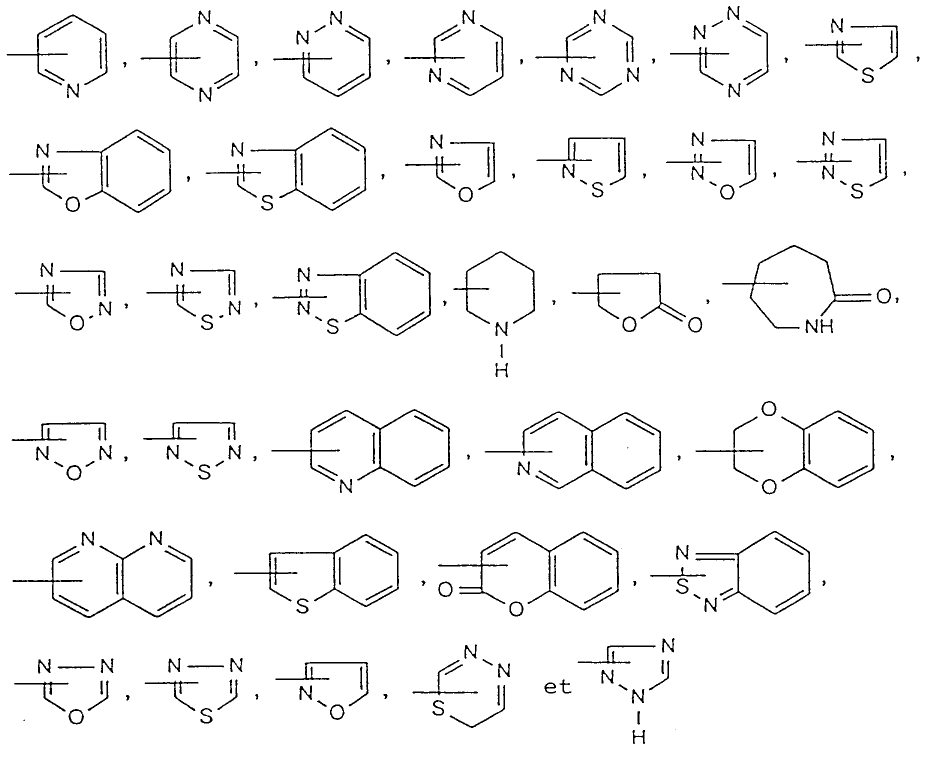 et est non substitué ou porte un, deux, trois ou quatre substituants choisis parmi les halogènes, les groupes alkyle en C1-C6, halogénoalkyle en C1-C4 contenant un à neuf atomes d'halogènes, alcoxy en C1-C4, halogénoalcoxy en C1-C4 contenant de un à neuf atomes d'halogènes, nitro, cyano, (alcoxy en C1-C4)carbonyle, di-(alkyle en C1-C4)amino, phényle, benzyle, pyridyle, thiényle et les groupes phényle, benzyle, pyridyle ou thiényle eux-mêmes monosubstitués par un halogène, un groupe alkyle en C1-C4, halogénoalkyle en C1-C4 contenant de un à neuf atomes d'halogènes, alcoxy en C1-C4, nitro ou cyano.

7. Un composé selon revendication 6, répondant à la formule I dans laquelle le radical A, relié à X par l'un de ses atomes de carbone, est choisi parmi les suivants : 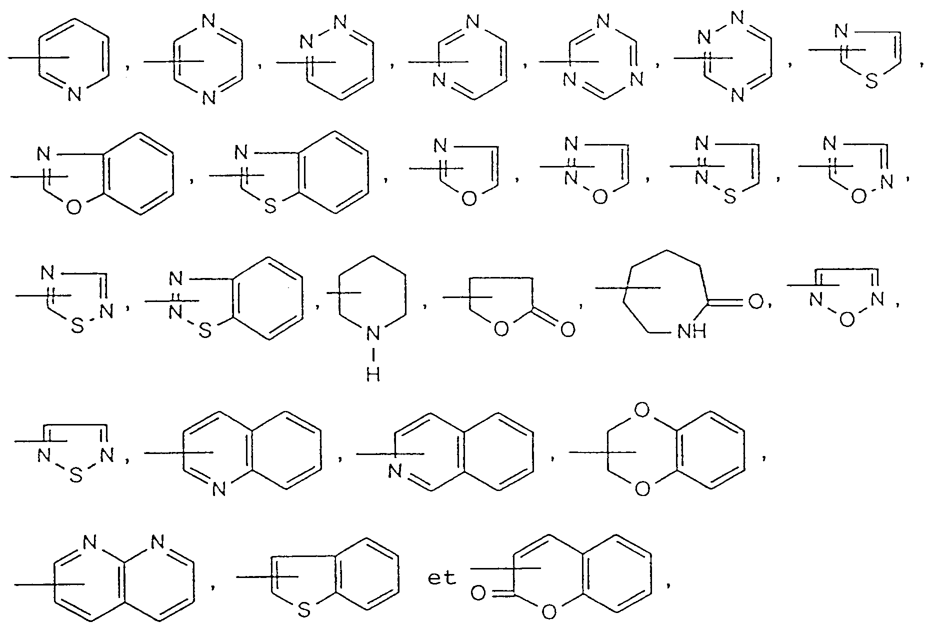 et est non substitué ou porte un ou deux substituants choisis parmi les halogènes, les groupes alkyle en C1-C6, halogénoalkyle en C1-C4 contenant de un à neuf atomes d'halogènes, alcoxy, (alcoxy en C1-C4)carbonyle, di- (alkyle en C1-C4)amino, phényle, benzyle, pyridyle, thiényle, et les groupes phényle, benzyle, pyridyle ou thiényle eux-mêmes monosubstitués par un halogène, un groupe alkyle en C1-C4, halogénoalkyle en C1-C4 contenant de un à neuf atomes d'halogènes, alcoxy en C1-C4, nitro ou cyano.

8. Un composé selon revendication 6, répondant à la formule I dans laquelle le radical A, relié à X par l'intermédiaire de l'un de ses atomes de carbone, est choisi parmi les suivants : 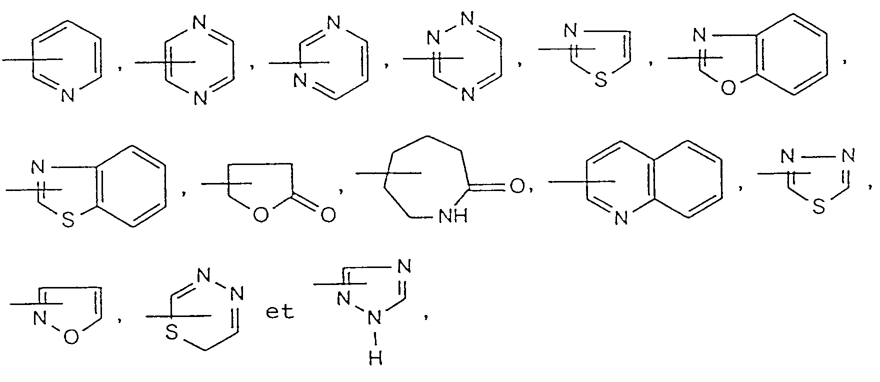 et est non substitué ou porte un, deux ou trois substituants choisis parmi les halogènes, les groupes alkyle en C1-C6, nitro, phényle, pyridyle et thiényle.

9. Un composé selon revendication 6, répondant à la formule I dans laquelle le radical A est choisi parmi les radicaux pyrid-2-yle, pyrid-3-yle, pyrimidin-2-yle, pyrazin-2-yle, thiazol-2-yle, isoxazyl-3-yle, 1,3,4-oxadiazol-2-yle, 1,3,4-thiadiazol-2-yle, benzoxazol-2-yle, benzothiazol-2-yle et quinoléin-3-yle, et est non substitué ou porte un substituant halogéno, méthyle, pyridyle ou thiényle.

10. Un composé selon la revendication 9, répondant à la formule I dans laquelle le radical A est un radical pyrid-2-yle non substitué ou thiazol-2-yle non substitué.

11. Un composé selon revendication 9, répondant à la formule I dans laquelle R₁ représente l'hydrogène, R₂ l'hydrogène, X représente NH ou S et le radical A est choisi parmi les radicaux pyrid-2-yle, pyrid-3-yle, pyrimidin-2-yle, pyrazin-2-yle, thiazol-2-yle, isoxazyl-3-yle, 1,3,4-oxadiazol-2-yle, 1,3,4-thiadiazol-2-yle, benzoxazol-2-yle, benzothiazol-2-yle et quinoléin-3-yle, et est non substitué ou porte un substituant halogéno, méthyle, pyridyle ou thiényle.

12. Un composé selon revendication 11, répondant à la formule I dans laquelle R₁ représente l'hydrogène, R₂ l'hydrogène, X représente NH et le radical A est un radical pyrid-2-yle non substitué ou thiazol-2-yle non substitué.

13. Un composé selon revendication 12, de formule

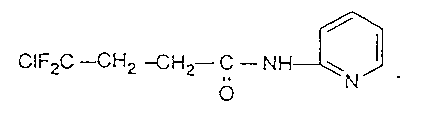

14. Un composé selon revendication 11, de formule

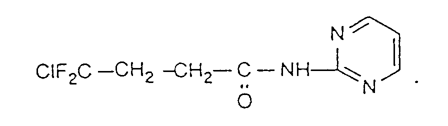

15. Un composé selon revendication 11, de formule

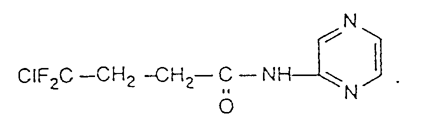

16. Un composé selon revendication 11, de formule

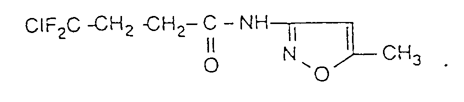

17. Un composé selon revendication 11, de formule

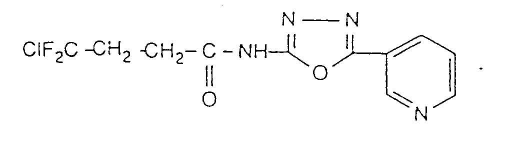

18. Un composé selon revendication 12, de formule

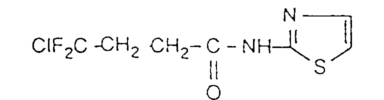

19. Procédé pour la préparation d'un composé selon revendication 1, répondant à la formule I, à l'état libre ou à l'état de sel, caractérisé en ce que
a) on fait réagir un composé de formule 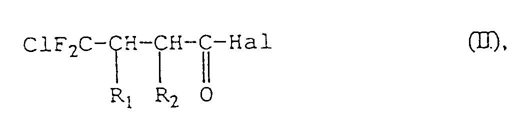 dans laquelle R₁ et R₂ ont les significations indiquées en référence à la formule I et Hal représente un halogène, de préférence le chlore ou le brome, en présence d'une base, avec un composé de formule
HX-A (III),
dans laquelle A et X ont les significations indiquées en référence à la formule I, ou avec l'un de ses sels, ou bien
b) on fait réagir un composé de formule 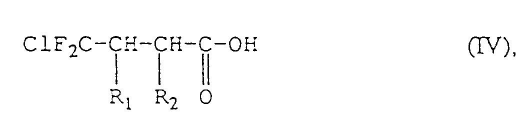 dans laquelle R₁ et R₂ ont les significations indiquées en référence à la formule I en présence d'un agent de condensation, avec un composé de formule III ou l'un de ses sels, ou bien
c) on fait réagir un composé de formule 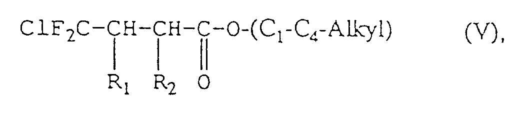 dans laquelle R₁ et R₂ ont les significations indiquées en référence à la formule I avec un composé de formule III ou l'un de ses sels,
et si on le désire, dans chaque cas, on convertit un composé de formule I obtenu comme décrit ci-dessus à l'état libre, en un sel ou un sel d'un composé de formule I obtenu comme décrit ci-dessus, en le composé libre de formule I ou en un autre sel.

20. Produit parasiticide, caractérisé en ce qu'il contient au moins un composé selon revendication 1 répondant à la formule I, à l'état libre ou à l'état de sel acceptable pour les applications agrochimiques, en tant que substance active, et au moins un produit auxiliaire.

21. Produit selon revendication 20, pour la lutte contre les insectes et/ou les arachnides.

22. Procédé pour la préparation d'un produit selon revendication 20, caractérisé en ce que l'on mélange intimement la substance active avec le ou les produits auxiliaires.

23. Utilisation d'un composé selon revendication 1, répondant à la formule I, à l'état libre ou à l'état de sel acceptable pour des applications agrochimiques, ou d'un produit selon revendication 20, pour la lutte contre les parasites.

24. Utilisation selon revendication 23, pour la lutte contre les insectes et/ou les arachnides.

25. Utilisation selon revendication 23, pour le traitement des semences.

26. Procédé pour combattre les parasites, caractérisé en ce que l'on applique en tant que substance active un composé selon revendication 1 répondant à la formule I, à l'état libre ou à l'état de sel convenant pour les applications agrochimiques, sur les parasites et/ou leur habitat.

27. Procédé selon revendication 26 pour la lutte contre les insectes et/ou les arachnides.

28. Procédé selon revendication 26 pour la protection des semences, caractérisé en ce que l'on traite les semences ou le sillon de semailles.

29. Semences traitées par le procédé décrit dans la revendication 28.
